# EUROPEAN PATENT APPLICATION

(11) **EP 4 317 438 A1**
(43) Date of publication of application: **07.02.2024**
(21) Application number: 22780213.9
(22) Date of filing: 17.03.2022
(51) Int. Cl.: C12N 15/53, C12N 1/15, C12N 1/19, C12N 1/21, C12N 5/10, C12N 9/04, C12N 9/10, C12N 15/63, C12P 7/42

(54) **RECOMBINANT POLYPEPTIDE HAVING ACYL-COA COMPOUND REDUCING ACTIVITY**

(30) Priority: 30.03.2021 JP 2021057313
(71) Applicant: Asahi Kasei Kabushiki Kaisha, Tokyo 1000006 (JP)
(72) Inventor: ISAKA Koji, Tokyo 100-0006 (JP); MIYATAKE Ryo, Tokyo 100-0006 (JP); KATAYAMA Sayaka, Tokyo 100-0006 (JP)
(74) Representative: D Young & Co LLP
(86) International application number: PCT/JP2022/012463
(87) International publication number: WO 2022/209994

(57) **Abstract**

Provided are a recombinant polypeptide having an excellent acyl-CoA compound reducing activity, and a production method of an aliphatic compound using the recombinant polypeptide.

A recombinant polypeptide has (a) an amino acid sequence A having a sequence identity of 60% or higher, 65% or higher, 70% or higher, 75% or higher, 80% or higher, 85% or higher, 88% or higher, 90% or higher, 93% or higher, 95% or higher, 97% or higher, 98% or higher, or 99% or higher with an amino acid sequence set forth in SEQ ID NO: 1; (b) a substitution of at least one amino acid at a position corresponding to a substrate-binding site of a polypeptide having an amino acid sequence set forth in SEQ ID NO: 1 in the amino acid sequence A; and (c) a reducing activity R of converting CoA thioester of an acyl-CoA compound into an aldehyde group, in which the reducing activity R includes (c-1) a reducing activity R1 of converting adipyl-CoA into 5-formylpentanoic acid in one step; and (c-2) a reducing activity R2 of converting succinyl-CoA into succinic semialdehyde.

## Description

### Technical Field

The present invention relates to a recombinant polypeptide having an acyl-CoA compound reducing activity, and a production method of an aliphatic compound using the peptide.

### Background Art

Due to weather disasters or climate change accompanying global warming in recent years, there is an increasing need for sustainable innovation aiming at symbiosis with the global environment and environmental conservation. Among them, a bioprocess, which is a substance production process in which a renewable raw material can be used and a biological reaction is used, has been greatly expected. So far, fermentation production processes for various chemical products have been developed. As an example, a fermentation production method using microorganisms has been studied for production of polyols used for a polyurethane raw material, a polyester raw material, a plasticizer raw material, a pharmaceutical intermediate, and the like (Patent Literatures 1, 2, and 8).

Examples of a compound having other reported examples of the fermentation production process include amine compounds containing an amino group. For example, 1,5-diaminopentane and 1,6-diaminohexane are fermentatively producible monomer compounds that are expected as raw materials for polymers (Patent Literatures 3 to 7).

Examples of a fermentation production pathway of a C6 monomer compound, 1,6-hexanediol, 6-aminocaproic acid, and 1,6-diaminohexane include adipic acid fermentation pathways (Non Patent Literatures 1 and 2). As an application of the adipic acid fermentation pathway, a pathway including a reaction of converting adipyl-CoA into 5-formylpentanoic acid through one or a plurality of steps using an aldehyde dehydrogenase, and an existing enzyme that can be a candidate for catalyzing the reaction have been found (Patent Literatures 4, 5, and 9). However, the availability of the existing enzyme in the enzyme reaction of the pathway is merely shown, and it is not shown that the enzyme that catalyzes the reaction actually exists. It has also been proposed to utilize butyl aldehyde dehydrogenase or succinic semialdehyde dehydrogenase for the synthesis of adipic semialdehyde from adipyl-CoA (Non Patent Literatures 3 and 4), but the product amount is not sufficient. The reason why a sufficient amount of product cannot be obtained is considered to be that the enzyme activity for each adipyl-CoA is low. In addition, succinic semialdehyde dehydrogenase is an enzyme important for growth of an organism, and has a high activity for succinyl-CoA which is an intermediate of the adipic acid pathway. Therefore, in order to obtain a sufficient amount of product, an enzyme having a low activity for succinyl-CoA has been desired.

### Citation List

### Patent Literature

Patent Literature 1: WO 2019/159831 A
Patent Literature 2: JP 2018-046843 A
Patent Literature 3: JP 2012-188407 A
Patent Literature 4: JP 2012-525856 A
Patent Literature 5: JP 2016-538870 A
Patent Literature 6: JP 2016-501031 A
Patent Literature 7: JP 2017-533734 A
Patent Literature 8: JP 2016-533162 A
Patent Literature 9: JP 2011-512868 A

### Non-Patent Literature

Non-Patent Literature 1: Yu, et. al. Direct biosynthesis of adipic acid from a synthetic pathway in recombinant Escherichia coli, Biotechnology and Bioengineering, Wiley Periodicals, Inc., 2014, vol. 111, pp. 2580.
Non-Patent Literature 2: Yu Zhou, et. al. Biosynthesis of adipic acid by a highly efficient induction-free system in Escherichia Coli, Journal of Biotechnology, Elsevier, 2020, 8, pp. 314-315
Non-Patent Literature 3: Cheong, et. al. Energy- and carbon-efficient synthesis of functionalized small molecules in bacteria using non-decarboxylative Claisen condensation reactions, Nature biotechnology, 2016
Non-Patent Literature 4: Turk et. al. Metabolic Engineering toward Sustainable Production of Nylon-6, ACS Synth. Biol. 2016, 5, pp. 65-73

### Summary of Invention

### Technical Problem

An object of the present invention is to provide a novel recombinant polypeptide having an acyl-CoA compound reducing activity. Further, another object of the present invention is to provide a novel production method of an aliphatic compound using the recombinant polypeptide.

### Solution to Problem

As a result of intensive studies to solve the above problems, the present inventors have found that a recombinant polypeptide capable of efficiently producing a target aliphatic compound is obtained by introducing a specific mutation into a wild-type succinic semialdehyde dehydrogenase. That is, the present invention is as follows:
[1] A recombinant polypeptide having:
   (a) an amino acid sequence A having a sequence identity of 60% or higher, 65% or higher, 70% or higher, 75% or higher, 80% or higher, 85% or higher, 88% or higher, 90% or higher, 93% or higher, 95% or higher, 97% or higher, 98% or higher, or 99% or higher with an amino acid sequence set forth in SEQ ID NO: 1;
   (b) a substitution of at least one amino acid at a position corresponding to a substrate-binding site of a polypeptide having an amino acid sequence set forth in SEQ ID NO: 1 in the amino acid sequence A; and
   (c) a reducing activity R of converting CoA thioester of an acyl-CoA compound into an aldehyde group,
      in which the reducing activity R includes:
      (c-1) a reducing activity R1 of converting adipyl-CoA into 5-formylpentanoic acid in one step; and
      (c-2) a reducing activity R2 of converting succinyl-CoA into succinic semialdehyde;
[2] The recombinant polypeptide according to [1], in which the reducing activity R2 of (c-2) is lower than a reducing activity R2' of the polypeptide having the amino acid sequence set forth in SEQ ID NO: 1 that converts succinyl-CoA into succinic semialdehyde;
[3] The recombinant polypeptide according to [1] or [2], in which the recombinant polypeptide is selected from the group of succinic semialdehyde dehydrogenase (EC 1.2.1.76);
[4] The recombinant polypeptide according to any one of [1] to [3], in which the recombinant polypeptide is derived from a microorganism of the genus *Clostridium;*
[5] The recombinant polypeptide according to any one of [1] to [4], in which the position corresponding to the substrate-binding site of the polypeptide having the amino acid sequence set forth in SEQ ID NO: 1 in (b) is a position corresponding to positions 75, 78, 79, 245, 250, 252, 405, 406, 410, 418, 419, 420, and 926 based on the amino acid sequence set forth in SEQ ID NO: 1;
[6] The recombinant polypeptide according to any one of [1] to [5], in which the reducing activity R1 of (c-1) is improved compared to a reducing activity R1' of the polypeptide having the amino acid sequence set forth in SEQ ID NO: 1 that converts adipyl-CoA into 5-formylpentanoic acid in one step;
[7] The recombinant polypeptide according to any one of [1] to [6], in which at least two amino acids at the positions corresponding to the substrate-binding site of the polypeptide having the amino acid sequence set forth in SEQ ID NO: 1 are substituted in the amino acid sequence A;
[8] The recombinant polypeptide according to any one of [1] to [7], in which in the amino acid sequence A, at least one of the following (i) to (xii) is satisfied based on the amino acid sequence set forth in SEQ ID NO: 1:
   (i) an amino acid residue at a position corresponding to position 75 is substituted with any of alanine and phenylalanine;
   (ii) an amino acid residue at a position corresponding to position 78 is substituted with any of alanine and phenylalanine;
   (iii) an amino acid residue at a position corresponding to position 79 is substituted with any of alanine and phenylalanine;
   (iv) an amino acid residue at a position corresponding to position 245 is substituted with any of alanine, phenylalanine, arginine, glutamic acid, serine, asparagine, glutamine, glycine, leucine, and tryptophan;
   (v) an amino acid residue at a position corresponding to position 250 is substituted with any of alanine, phenylalanine, lysine, arginine, histidine, threonine, asparagine, glutamine, glycine, leucine, valine, proline, and tryptophan;
   (vi) an amino acid residue at a position corresponding to position 252 is substituted with any of alanine and phenylalanine;
   (vii) an amino acid residue at a position corresponding to position 405 is substituted with any of alanine, phenylalanine, lysine, histidine, glutamic acid, serine, asparagine, glutamine, leucine, valine, tyrosine, proline, and tryptophan;
   (viii) an amino acid residue at a position corresponding to position 406 is substituted with any of alanine and phenylalanine;
   (ix) an amino acid residue at a position corresponding to position 410 is substituted with phenylalanine;
   (x) an amino acid residue at a position corresponding to position 418 is substituted with any of alanine and phenylalanine;
   (xi) an amino acid residue at a position corresponding to position 419 is substituted with any of alanine and phenylalanine; and
   (xii) an amino acid residue at a position corresponding to position 420 is substituted with any of alanine and phenylalanine;
[9] The recombinant polypeptide according to any one of [1] to [8], in which in the amino acid sequence A, at least one of the following (i) to (xii) is satisfied based on the amino acid sequence set forth in SEQ ID NO: 1:
   (i) an amino acid residue at a position corresponding to position 75 is substituted with any of alanine and phenylalanine;
   (ii) an amino acid residue at a position corresponding to position 78 is substituted with any of alanine and phenylalanine;
   (iii) an amino acid residue at a position corresponding to position 79 is substituted with alanine;
   (iv) an amino acid residue at a position corresponding to position 245 is substituted with any of alanine, phenylalanine, arginine, glutamic acid, serine, asparagine, glutamine, glycine, leucine, and tryptophan;
   (v) an amino acid residue at a position corresponding to position 250 is substituted with any of alanine, phenylalanine, lysine, arginine, histidine, threonine, asparagine, glutamine, glycine, leucine, valine, proline, and tryptophan;
   (vi) an amino acid residue at a position corresponding to position 252 is substituted with any of alanine and phenylalanine;
   (vii) an amino acid residue at a position corresponding to position 405 is substituted with any of alanine, phenylalanine, lysine, histidine, glutamic acid, serine, asparagine, glutamine, leucine, valine, tyrosine, proline, and tryptophan;
   (viii) an amino acid residue at a position corresponding to position 406 is substituted with any of alanine and phenylalanine;
   (ix) an amino acid residue at a position corresponding to position 410 is substituted with phenylalanine;
   (x) an amino acid residue at a position corresponding to position 418 is substituted with alanine;
   (xi) an amino acid residue at a position corresponding to position 419 is substituted with alanine; and
   (xii) an amino acid residue at a position corresponding to position 420 is substituted with alanine;
[10] The recombinant polypeptide according to any one of [1] to [9], in which in the amino acid sequence A, at least one of the following (i) to (xii) is satisfied based on the amino acid sequence set forth in SEQ ID NO: 1:
   (i) an amino acid residue at a position corresponding to position 75 is substituted with any of alanine and phenylalanine;
   (ii) an amino acid residue at a position corresponding to position 78 is substituted with phenylalanine;
   (iii) an amino acid residue at a position corresponding to position 79 is substituted with any of alanine and phenylalanine;
   (iv) an amino acid residue at a position corresponding to position 245 is substituted with any of arginine, glutamine, glycine, serine, and tryptophan;
   (v) an amino acid residue at a position corresponding to position 250 is substituted with any of alanine, phenylalanine, leucine, and proline;
   (vi) an amino acid residue at a position corresponding to position 252 is substituted with any of alanine and phenylalanine;
   (vii) an amino acid residue at a position corresponding to position 405 is substituted with any of alanine, phenylalanine, lysine, histidine, glutamic acid, serine, glutamine, tyrosine, and tryptophan;
   (viii) an amino acid residue at a position corresponding to position 406 is substituted with any of alanine and phenylalanine;
   (ix) an amino acid residue at a position corresponding to position 410 is substituted with phenylalanine;
   (x) an amino acid residue at a position corresponding to position 418 is substituted with any of alanine and phenylalanine;
   (xi) an amino acid residue at a position corresponding to position 419 is substituted with any of alanine and phenylalanine; and
   (xii) an amino acid residue at a position corresponding to position 420 is substituted with any of alanine and phenylalanine;
[11] The recombinant polypeptide according to any one of [1] to [10], in which in the amino acid sequence A, at least one of the following is satisfied based on the amino acid sequence set forth in SEQ ID NO: 1:
   (iv) an amino acid residue at a position corresponding to position 245 is substituted with any of arginine, serine, and tryptophan;
   (v) an amino acid residue at a position corresponding to position 250 is substituted with leucine; and
   (vii) an amino acid residue at a position corresponding to position 405 is substituted with any of lysine, histidine, serine, glutamine, tyrosine, and tryptophan;
[12] The recombinant polypeptide according to any one of [1] to [11], in which in the amino acid sequence A, any one of the following is satisfied based on the amino acid sequence set forth in SEQ ID NO: 1,
   the substitution is made at any one of:
   · a position corresponding to position 245 and a position corresponding to position 250;
   · a position corresponding to position 245 and a position corresponding to position 405;
   · a position corresponding to position 250 and a position corresponding to position 405; and
   · a position corresponding to position 405 and a position corresponding to position 418;
[13] The recombinant polypeptide according to any one of [1] to [12], in which in the amino acid sequence A, any one of the following is satisfied based on the amino acid sequence set forth in SEQ ID NO: 1:
   - an amino acid residue at a position corresponding to 245 position is arginine and an amino acid at a position corresponding to 250 position is leucine;
   - an amino acid residue at a position corresponding to 245 position is arginine and an amino acid at a position corresponding to 405 position is tyrosine;
   - an amino acid residue at a position corresponding to 245 position is tryptophan and an amino acid at a position corresponding to 405 position is tyrosine;
   - an amino acid residue at a position corresponding to 245 position is arginine and an amino acid at a position corresponding to 405 position is histidine;
   - an amino acid residue at a position corresponding to 250 position is leucine and an amino acid at a position corresponding to 405 position is tyrosine; and
   - an amino acid residue at a position corresponding to 418 position is alanine and an amino acid at a position corresponding to 405 position is phenylalanine;
[14] A DNA encoding the recombinant polypeptide according to any one of [1] to [13];
[15] A recombinant plasmid containing the DNA according to [14];
[16] A recombinant microorganism into which the DNA according to [14] is introduced;
[17] The recombinant microorganism according to [16], the recombinant microorganism has an adipyl-CoA production ability;
[18] A production method of a target compound, the production method including converting adipyl-CoA into 5-formylpentanoic acid in the presence of the recombinant polypeptide according to any one of [1] to [13], or a culture of the recombinant microorganism according to [16] or [17] and/or an extract of the culture,
   in which the target compound is selected from the group consisting of 5-formylpentanoic acid, 6-aminocaproic acid, 1,6-diaminohexane, 6-hydroxyhexanoic acid, hexanedial, 6-aminohexanal, 6-hydroxyhexanal, and 1,6-hexanediol;
[19] The production method according to [18], further including converting the 5-formylpentanoic acid into 6-aminocaproic acid,
   in which the target compound is 6-aminocaproic acid;
[20] The production method according to [18], further including converting the 5-formylpentanoic acid into 6-hydroxyhexanoic acid,
   in which the target compound is 6-hydroxyhexanoic acid;
[21] The production method according to [18], further including:
   i) converting the 5-formylpentanoic acid into 6-hydroxyhexanoic acid,
      converting the 6-hydroxyhexanoic acid into 6-hydroxyhexanal, and
      converting the 6-hydroxyhexanal into 1,6-hexanediol; or
   ii) converting the 5-formylpentanoic acid into hexanedial,
      converting the hexanedial into 6-hydroxyhexanal, and
      converting the 6-hydroxyhexanal into 1,6-hexanediol,
      in which the target compound is 1,6-hexanediol;
[22] The production method according to [18], further including:
   i) converting the 5-formylpentanoic acid into 6-aminocaproic acid,
      converting the 6-aminocaproic acid into 6-aminohexanal, and
      converting the 6-aminohexanal into 1,6-diaminohexane; or
   ii) converting the 5-formylpentanoic acid into hexanedial,
      converting the hexanedial into 6-aminohexanal, and
      converting the 6-aminohexanal into 1,6-diaminohexane,
      in which the target compound is 1,6-diaminohexane;
[23] The production method according to [19] or [22], in which the converting of the 5-formylpentanoic acid into 6-aminocaproic acid, the converting of the hexanedial into 6-aminohexanal, and the converting of the 6-aminohexanal into 1,6-diaminohexane are catalyzed by an enzyme selected from the group consisting of:
   · 4-aminobutanoate-2-oxoglutarate transaminase (EC 2.6.1.19) ;
   · putrescine-2-oxoglutarate transaminase (EC 2.6.1.82) ;
   · 4-aminobutanoate-pyruvate transaminase (EC 2.6.1.96); and
   · putrescine-pyruvate transaminase (EC 2.6.1.113);
[24] The production method according to [23], in which the enzyme is derived from a microorganism selected from the group consisting of the genus *Vibrio* and the genus *Escherichia;*
[25] The production method according to [21] or [22], in which the converting of the 6-hydroxyhexanoic acid into 6-hydroxyhexanal, the converting of the 5-formylpentanoic acid into hexanedial, and the converting of the 6-aminocaproic acid into 6-aminohexanal are catalyzed by an enzyme belonging to a carboxylate reductase (EC 1.2.1.30) ;
[26] The production method according to [25], in which the carboxylate reductase is derived from a microorganism selected from the group consisting of the genus *Mycobacterium* and the genus *Nocardia;*
[27] The production method according to [20] or [21], in which the converting of the 5-formylpentanoic acid into 6-hydroxyhexanoic acid, the converting of the hexanedial into 6-hydroxyhexanal, and the converting of the 6-hydroxyhexanal into 1,6-hexanediol are catalyzed by an enzyme selected from the group consisting of:
   · alcohol dehydrogenase (EC 1.1.1.1); and
   · alcohol dehydrogenase (EC 1.1.1.2);
[28] The production method according to [27], in which the alcohol dehydrogenase is derived from a microorganism selected from the group consisting of the genus *Escherichia* and the genus *Bacillus;*
[29] A recombinant microorganism having:
   a pathway for producing at least one selected from the group consisting of 5-formylpentanoic acid, 6-aminocaproic acid, 1,6-diaminohexane, 6-hydroxyhexanoic acid, hexanedial, 6-aminohexanal, 6-hydroxyhexanal, and 1,6-hexanediol; and
   an exogenous nucleic acid sequence encoding an enzyme having an amino acid sequence set forth in SEQ ID NO: 1 or an enzyme having an amino acid sequence having a sequence identity of 60% or higher, 65% or higher, 70% or higher, 75% or higher, 80% or higher, 85% or higher, 88% or higher, 90% or higher, 93% or higher, 95% or higher, 97% or higher, 98% or higher, or 99% or higher with the amino acid sequence set forth in SEQ ID NO: 1 and having a reducing activity R of converting CoA thioester of an acyl-CoA compound into an aldehyde group; and
[30] A recombinant polypeptide having:
   (a) an amino acid sequence A having a sequence identity of 85% or higher, 88% or higher, 90% or higher, 93% or higher, 95% or higher, 97% or higher, 98% or higher, or 99% or higher with an amino acid sequence set forth in SEQ ID NO: 1;
   (b) a substitution of at least one amino acid at a position corresponding to a substrate-binding site of a polypeptide having an amino acid sequence set forth in SEQ ID NO: 1 in the amino acid sequence A; and
   (c) a reducing activity R of converting CoA thioester of an acyl-CoA compound into an aldehyde group.

### Advantageous Effects of Invention

According to the present invention, it is possible to provide a novel recombinant polypeptide having an acyl-CoA compound reducing activity and a novel production method of an aliphatic compound using the recombinant polypeptide.

### Brief Description of Drawings

Fig. 1 is a diagram showing an amino acid sequence (SEQ ID NO: 1) of a wild-type succinic semialdehyde dehydrogenase.
Fig. 2 is a diagram showing an amino acid sequence (SEQ ID NO: 2) encoding a wild-type succinic semialdehyde dehydrogenase.
Fig. 3A is a diagram showing an amino acid sequence (SEQ ID NO: 121) of an aldehyde dehydrogenase derived from *Natronincola ferrireducens.*
Fig. 3B is a diagram showing the alignment results of the amino acid sequence of SEQ ID NO: 1 and the amino acid sequence (SEQ ID NO: 121) of an aldehyde dehydrogenase derived from *Natronincola ferrireducens* having a sequence identity of 66% with SEQ ID NO: 1.
Fig. 3C is a diagram showing a part of the alignment results of the amino acid sequence of SEQ ID NO: 1 and the amino acid sequence (SEQ ID NO: 121) of an aldehyde dehydrogenase derived from *Natronincola ferrireducens* having a sequence identity of 66% with SEQ ID NO: 1.
Fig. 4 is a diagram showing a reaction pathway involving a recombinant polypeptide according to the present invention.
Fig. 5 is a schematic diagram of a plasmid of pSK000.
Fig. 6 is a diagram showing synthesis pathways of various compounds starting from adipyl-CoA.
Fig. 7 is a diagram showing mutation sites and amino acids after mutation of a modified enzyme, and enzyme activities (substrate consumption rate per unit amount of enzyme per unit time (µmol/min/µg enzyme)) for respective substrates (adipyl-CoA and succinyl-CoA).
Fig. 8 is a diagram showing mutation sites and amino acids after mutation of a modified enzyme containing a plurality of mutations, and enzyme activities (substrate consumption rate per unit amount of enzyme per unit time (µmol/min/µg enzyme)) for respective substrates (adipyl-CoA and succinyl-CoA).

### Mode for Carrying Out the Invention

Hereinafter, modes for carrying out the present invention will be specifically described. It should be noted that the present invention is not limited to the following embodiments, and various modifications can be made within the scope of the gist of the present invention. In addition, unless otherwise specified, genetic manipulations such as acquisition of DNA, preparation of a vector, and transformation described in the present specification can be performed by the methods described in known documents such as Molecular Cloning 4th Edition (Cold Spring Harbor Laboratory Press, 2012), Current Protocols in Molecular Biology (Greene Publishing Associates and Wiley-Interscience), and genetic engineering experimental note (Takaaki Tamura, YODOSHA CO., LTD.). It should be noted that, in the present specification, unless otherwise specified, nucleotide sequences are described in the 5' to 3' directions. In the present specification, a range of numerical values indicated by "to" represents a range including numerical values described before and after "to" as the minimum value and the maximum value, respectively. In the range of the numerical values described in stages in the present specification, an upper limit value or a lower limit value of a range of numerical values of a certain stage can be arbitrarily combined with an upper limit value or a lower limit value of a range of numerical values of another stage.

A recombinant polypeptide according to the present invention is a polypeptide that has been modified so as to have an excellent acyl-CoA compound reducing activity. More specifically, the recombinant polypeptide according to the present invention has been modified so as to have an improved acyl-CoA compound reducing activity as compared with a wild-type acyl-CoA compound reductase. In the present specification, the terms "recombinant polypeptide" and "modified enzyme" are used interchangeably.

Regarding the recombinant polypeptide according to the present invention, the "acyl-CoA compound reducing activity" is a reducing activity R of converting CoA thioester of an acyl-CoA compound into an aldehyde group. Typically, CoA thioester of an acyl-CoA compound is first converted into a carboxyl group, and the carboxyl group is then further converted into an aldehyde group. Here, "CoA" means a coenzyme A (hereinafter, also referred to as a "coenzyme A").

Further, regarding the recombinant polypeptide according to the present invention, the terms "acyl-CoA compound reductase", "acyl-CoA reductase", and "aldehyde dehydrogenase" are used interchangeably.

The "acyl-CoA compound" is a generic term for compounds in which various acyl groups are thioester-bonded to a terminal thiol group. Examples of the "acyl-CoA compound" include acetyl-CoA (where the acyl group is an acetyl group), succinyl-CoA (where the acyl group is a succinyl group), and adipyl-CoA (where the acyl group is an adipyl group).

In the present specification, regarding the "acyl-CoA compound", an acyl group has, for example, 2 to 10 carbons, preferably 2 to 6 carbons, and more preferably 4 to 6 carbons. More specifically, regarding the recombinant polypeptide according to the present invention, the "acyl-CoA compound" includes succinyl-CoA and adipyl-CoA.

The recombinant polypeptide according to the present invention has properties shown in the following (a) to (c):
(a) an amino acid sequence A having a sequence identity of 60% or higher with an amino acid sequence of a wild-type acyl-CoA compound reductase;
(b) a substitution of at least one amino acid at a position corresponding to a substrate-binding site of a wide-type acyl-CoA compound reductase in the amino acid sequence A; and
(c) a reducing activity R of converting CoA thioester of an acyl-CoA compound into an aldehyde group.

In a preferred embodiment, the recombinant polypeptide according to the present invention has:
(a) an amino acid sequence A having a sequence identity of 85% or higher, 88% or higher, 90% or higher, 93% or higher, 95% or higher, 97% or higher, 98% or higher, or 99% or higher with an amino acid sequence set forth in SEQ ID NO: 1;
(b) a substitution of at least one amino acid at a position corresponding to a substrate-binding site of a polypeptide having an amino acid sequence set forth in SEQ ID NO: 1 in the amino acid sequence A; and
(c) a reducing activity R of converting CoA thioester of an acyl-CoA compound into an aldehyde group.

The recombinant polypeptide according to the present invention has an amino acid sequence A having a certain percentage or more of a sequence identity with an amino acid sequence of a wild-type acyl-CoA compound reductase. The wild-type acyl-CoA compound reductase is preferably selected from the group of succinic semialdehyde dehydrogenase (coenzyme-A-dependent succinate-semialdehyde dehydrogenase) (EC 1.2.1.76). The origin of the wild-type acyl-CoA compound reductase is not limited, and can be obtained from, for example, a microorganism selected from the group consisting of the genus *Clostridium,* the genus *Candida,* and the genus *Escherichia.* The wild-type acyl-CoA compound reductase can be preferably obtained from a microorganism belonging to the genus *Clostridium.* The wild-type acyl-CoA compound reductase can be most preferably obtained from *Clostridium difficile.*

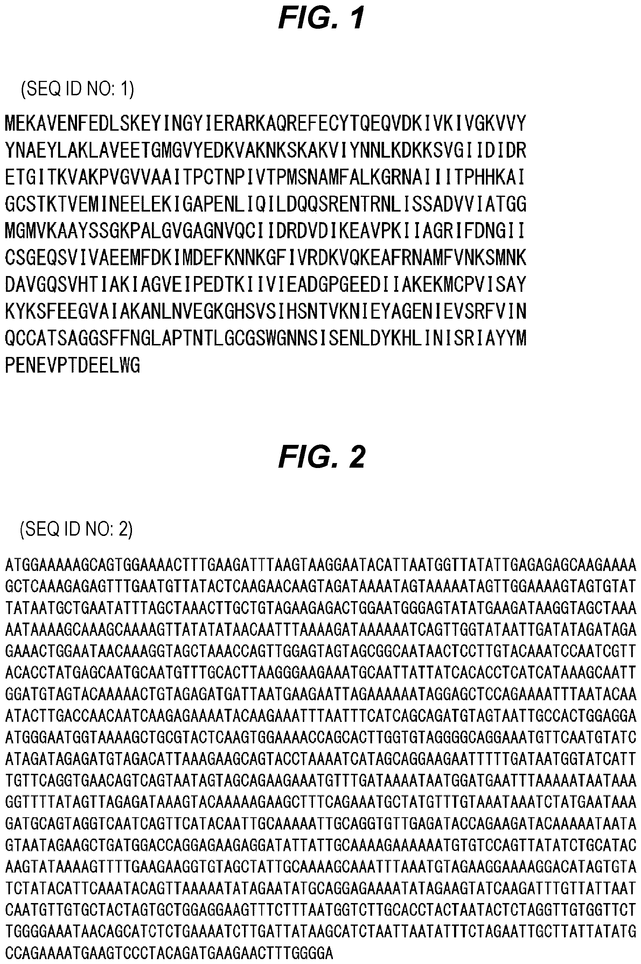

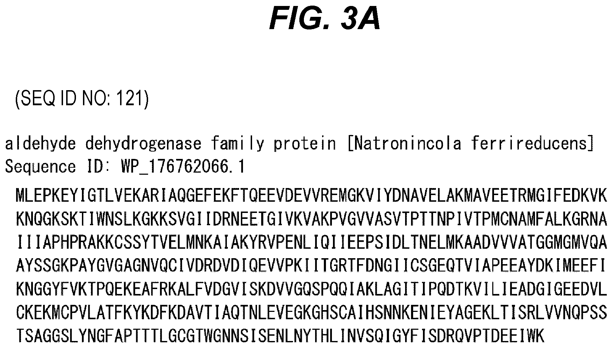

As an example of the wild-type acyl-CoA compound reductase, an amino acid sequence of an acyl-CoA compound reductase derived from *Clostridium difficile* is set forth in SEQ ID NO: 1 (Fig. 1). As another example of the wild-type acyl-CoA compound reductase, an amino acid sequence of a wild-type acyl-CoA compound reductase derived from *Natronincola ferrireducens* is set forth in SEQ ID NO: 121 (Fig. 3A). The enzyme has a sequence identity of 66% with the amino acid sequence set forth in SEQ ID NO: 1.

The "wild-type acyl-CoA compound reductase" is not limited to a polypeptide having the amino acid sequence set forth in SEQ ID NO: 1, and may be an amino acid sequence in which one or a plurality of amino acids are substituted, added, inserted, or deleted from the amino acid sequence set forth in SEQ ID NO: 1. In addition, one or a plurality of amino acids may be added to either or both of the N-terminus and the C-terminus of the polypeptide. Specifically, the "wild-type acyl-CoA compound reductase" includes a polypeptide having an amino acid sequence set forth in SEQ ID NO: 1, as well as a polypeptide having an amino acid sequence having a sequence identity of 60% or more with the amino acid sequence set forth in SEQ ID NO: 1.

In the present specification, the "wild-type acyl-CoA compound reductase" is a polypeptide having an amino acid sequence having an amino acid sequence having a sequence identity of 60% or more with the amino acid sequence set forth in SEQ ID NO: 1, and is a polypeptide capable of performing an aldehyde synthesis reaction using an acyl-CoA compound (specifically, acyl-CoA thioester) as a substrate, in the presence of nicotinamide adenine dinucleotide phosphate (NADPH). Therefore, the "acyl-CoA compound reductase activity" or "acyl-CoA compound reducing activity" more specifically means an activity capable of performing an aldehyde synthesis reaction using an acyl-CoA compound as a substrate in the presence of NADPH.

The recombinant polypeptide according to the present invention has an amino acid sequence A having a certain percentage or more of a sequence identity with an amino acid sequence of a wild-type acyl-CoA compound reductase. Specifically, the recombinant polypeptide according to the present invention has an amino acid sequence A having a sequence identity of 60% or more, 65% or more, 70% or more, 75% or more, 80% or more, 85% or more, 88% or more, 90% or more, 93% or more, 95% or more, 97% or more, 98% or more, or 99% or more with the amino acid sequence of the wild-type acyl-CoA compound reductase. Therefore, in an embodiment, the amino acid sequence A of the recombinant polypeptide according to the present invention has a sequence identity of 60% or more, 65% or more, 70% or more, 75% or more, 80% or more, 85% or more, 88% or more, 90% or more, 93% or more, 95% or more, 97% or more, 98% or more, or 99% or more with the amino acid sequence set forth in SEQ ID NO: 1.

Regarding the amino acid sequence, the "sequence identity" can be determined by a known method, and is obtained using, for example, an alignment search tool that can be used in Basic Local Alignment Search Tool (BLAST, https://blast.ncbi.nlm.nih.gov/Blast.cgi). The method for determining the sequence identity using this program will be understood by those skilled in the art by reference to the above website, for example, the "help" section. For sequence comparison, Blast2 sequence function of BLAST (registered trademark, omitted below) (Blastp) program can be used using default parameters (gap existence cost 11 and gap cost 1 per residue). Similarly, a "sequence identity" of a polynucleotide sequence can also be determined by a known method.

The gene of the wild-type acyl-CoA compound reductase that can be used in the present invention is, for example, a polynucleotide whose sequence information can be obtained from Genebank gene accession number NZ_CP019870.1 and which is set forth in SEQ ID NO: 2 (Fig. 2). The gene can be obtained from a *Clostridium difficile* strain by a general genetic engineering technique. That is, the gene can be obtained as a gene product amplified by PCR using a genomic DNA as a template, and can be used for expression in a host microorganism. In addition, a polynucleotide prepared by organic synthesis can also be used. In addition, alternative codons that ultimately translate to the same amino acid may also be utilized. Typically, a method for replacing the gene sequence in consideration of the codon usage frequency of the host microorganism is used.

Hereinafter, the present invention will be described in detail in the case where the wild-type acyl-CoA compound reductase is a polypeptide having an amino acid sequence set forth in SEQ ID NO: 1.

At least one amino acid at a position corresponding to a substrate-binding site of a polypeptide having an amino acid sequence set forth in SEQ ID NO: 1 is substituted in the amino acid sequence A. Preferably, at least two amino acids at the positions corresponding to the substrate-binding site of the polypeptide having the amino acid sequence set forth in SEQ ID NO: 1 are substituted in the amino acid sequence A.

Regarding the amino acid sequence A, the "position corresponding to the substrate-binding site of the polypeptide having the amino acid sequence set forth in SEQ ID NO: 1" is, for example, a position corresponding to positions 74, 75, 78, 79, 245, 250, 251, 252, 404, 405, 406, 407, 408, 409, 410, 411, 418, 419, 420, and 926 based on the amino acid sequence set forth in SEQ ID NO: 1. In the present embodiment, in the amino acid sequence A, based on the amino acid sequence set forth in SEQ ID NO: 1, among the amino acids at the position corresponding to positions 74, 75, 78, 79, 245, 250, 251, 252, 404, 405, 406, 407, 408, 409, 410, 411, 418, 419, 420, and 926, one or a plurality of amino acids are substituted. When the amino acid sequence A has the above substitution, the enzyme activity for the substrate can be adjusted. A method for specifying the substrate-binding site will be described below.

In a preferred embodiment, regarding the amino acid sequence A, the "position corresponding to the substrate-binding site of the polypeptide having the amino acid sequence set forth in SEQ ID NO: 1" is a position corresponding to positions 75, 78, 79, 245, 250, 252, 405, 406, 410, 418, 419, 420, and 926 based on the amino acid sequence set forth in SEQ ID NO: 1. Therefore, in the present embodiment, in the amino acid sequence A, based on the amino acid sequence set forth in SEQ ID NO: 1, among the amino acids at the position corresponding to positions 75, 78, 79, 245, 250, 252, 405, 406, 410, 418, 419, 420, and 926, one or a plurality of amino acids are substituted.

Regarding the amino acid sequence A, the term "the amino acid residue at the position corresponding to position X (X represents an integer of 1 or more) based on the amino acid sequence set forth in SEQ ID NO: 1" includes:
· an amino acid residue at a position corresponding to position X based on the amino acid sequence set forth in SEQ ID NO: 1 (case 1); and
· an amino acid residue at position X of the amino acid sequence set forth in SEQ ID NO: 1 (case 2).

Specifically, the term "amino acid residue at the position corresponding to positions 75, 78, 79, 245, 250, 252, 405, 406, 410, 418, 419, 420, and 926 based on the amino acid sequence set forth in SEQ ID NO: 1" includes both:
· an amino acid residue at a position corresponding to positions 75, 78, 79, 245, 250, 252, 405, 406, 410, 418, 419, 420, and 926 based on the amino acid sequence set forth in SEQ ID NO: 1; and
· an amino acid residue at positions 75, 78, 79, 245, 250, 252, 405, 406, 410, 418, 419, 420, and 926 of the amino acid sequence set forth in SEQ ID NO: 1.

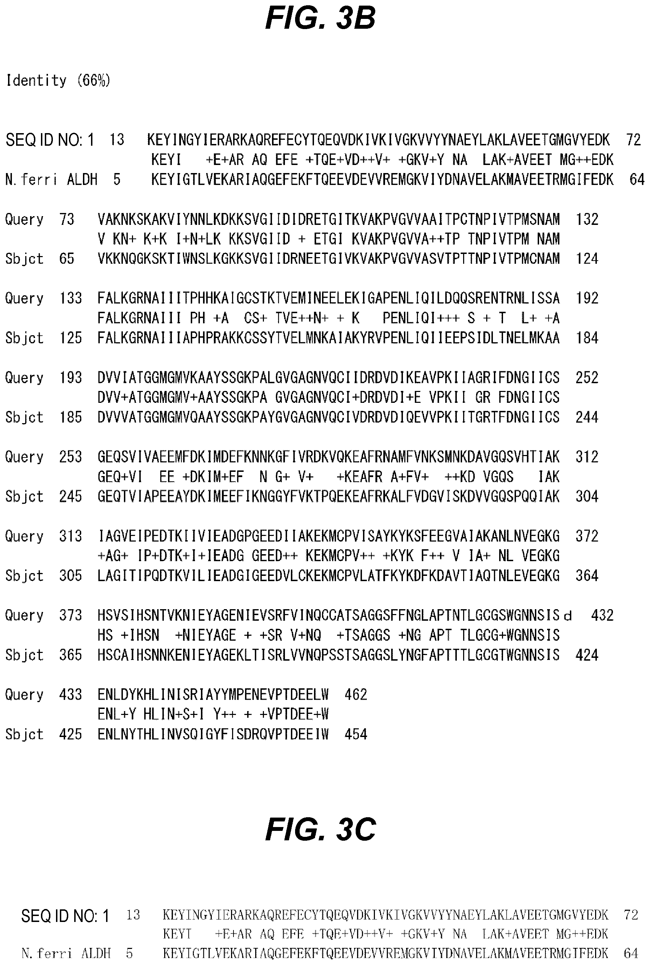

In the present invention, when an amino acid sequence as a reference (specifically, the amino acid sequence set forth in SEQ ID NO: 1, hereinafter referred to as a "reference sequence"), and a amino acid sequence to be compared (specifically, the amino acid sequence A) are aligned using BLAST (https://blast.ncbi.nlm.nih.gov/Blast.cgi) sequence analysis software, the "amino acid residue at the corresponding position" refers to an amino acid residue corresponding to a specific site in the reference sequence. For example, Fig. 3 illustrates the alignment results of SEQ ID NO: 1 and an amino acid sequence of an aldehyde dehydrogenase derived from *Natronincola ferrireducens* (having a sequence identity of 66% with SEQ ID NO: 1) when aligned using BLAST sequence analysis software. As illustrated in Fig. 3, in the amino acid sequence of an aldehyde dehydrogenase derived from *Natronincola ferrireducens,* an amino acid residue at a position corresponding to position 17 (asparagine residue in SEQ ID NO: 1) of the amino acid sequence set forth in SEQ ID NO: 1 is a glycine residue at position 9.

In one embodiment, in the recombinant polypeptide according to the present invention, in the amino acid sequence A, at least one of the following (i) to (xii) based on the amino acid sequence set forth in SEQ ID NO: 1 is satisfied:
(i) an amino acid residue at a position corresponding to position 75 is substituted with any of alanine and phenylalanine;
(ii) an amino acid residue at a position corresponding to position 78 is substituted with any of alanine and phenylalanine;
(iii) an amino acid residue at a position corresponding to position 79 is substituted with any of alanine and phenylalanine;
(iv) an amino acid residue at a position corresponding to position 245 is substituted with any of alanine, phenylalanine, arginine, glutamic acid, serine, asparagine, glutamine, glycine, leucine, and tryptophan;
(v) an amino acid residue at a position corresponding to position 250 is substituted with any of alanine, phenylalanine, lysine, arginine, histidine, threonine, asparagine, glutamine, glycine, leucine, valine, proline, and tryptophan;
(vi) an amino acid residue at a position corresponding to position 252 is substituted with any of alanine and phenylalanine;
(vii) an amino acid residue at a position corresponding to position 405 is substituted with any of alanine, phenylalanine, lysine, histidine, glutamic acid, serine, asparagine, glutamine, leucine, valine, tyrosine, proline, and tryptophan;
(viii) an amino acid residue at a position corresponding to position 406 is substituted with any of alanine and phenylalanine;
(ix) an amino acid residue at a position corresponding to position 410 is substituted with phenylalanine;
(x) an amino acid residue at a position corresponding to position 418 is substituted with any of alanine and phenylalanine;
(xi) an amino acid residue at a position corresponding to position 419 is substituted with any of alanine and phenylalanine; and
(xii) an amino acid residue at a position corresponding to position 420 is substituted with any of alanine and phenylalanine.

In a preferred embodiment, in the recombinant polypeptide according to the present invention, in the amino acid sequence A, at least one of the following (i) to (xii) based on the amino acid sequence set forth in SEQ ID NO: 1 is satisfied:
(i) an amino acid residue at a position corresponding to position 75 is substituted with any of alanine and phenylalanine;
(ii) an amino acid residue at a position corresponding to position 78 is substituted with any of alanine and phenylalanine;
(iii) an amino acid residue at a position corresponding to position 79 is substituted with alanine;
(iv) an amino acid residue at a position corresponding to position 245 is substituted with any of alanine, phenylalanine, arginine, glutamic acid, serine, asparagine, glutamine, glycine, leucine, and tryptophan;
(v) an amino acid residue at a position corresponding to position 250 is substituted with any of alanine, phenylalanine, lysine, arginine, histidine, threonine, asparagine, glutamine, glycine, leucine, valine, proline, and tryptophan;
(vi) an amino acid residue at a position corresponding to position 252 is substituted with any of alanine and phenylalanine;
(vii) an amino acid residue at a position corresponding to position 405 is substituted with any of alanine, phenylalanine, lysine, histidine, glutamic acid, serine, asparagine, glutamine, leucine, valine, tyrosine, proline, and tryptophan;
(viii) an amino acid residue at a position corresponding to position 406 is substituted with any of alanine and phenylalanine;
(ix) an amino acid residue at a position corresponding to position 410 is substituted with phenylalanine;
(x) an amino acid residue at a position corresponding to position 418 is substituted with alanine;
(xi) an amino acid residue at a position corresponding to position 419 is substituted with alanine; and
(xii) an amino acid residue at a position corresponding to position 420 is substituted with alanine.

In a more preferred embodiment, in the recombinant polypeptide according to the present invention, in the amino acid sequence A, at least one of the following (i) to (xii) based on the amino acid sequence set forth in SEQ ID NO: 1 is satisfied:
(i) an amino acid residue at a position corresponding to position 75 is substituted with any of alanine and phenylalanine;
(ii) an amino acid residue at a position corresponding to position 78 is substituted with phenylalanine;
(iii) an amino acid residue at a position corresponding to position 79 is substituted with any of alanine and phenylalanine;
(iv) an amino acid residue at a position corresponding to position 245 is substituted with any of arginine, glutamine, glycine, serine, and tryptophan;
(v) an amino acid residue at a position corresponding to position 250 is substituted with any of alanine, phenylalanine, leucine, and proline;
(vi) an amino acid residue at a position corresponding to position 252 is substituted with any of alanine and phenylalanine;
(vii) an amino acid residue at a position corresponding to position 405 is substituted with any of alanine, phenylalanine, lysine, histidine, glutamic acid, serine, glutamine, tyrosine, and tryptophan;
(viii) an amino acid residue at a position corresponding to position 406 is substituted with any of alanine and phenylalanine;
(ix) an amino acid residue at a position corresponding to position 410 is substituted with phenylalanine;
(x) an amino acid residue at a position corresponding to position 418 is substituted with any of alanine and phenylalanine;
(xi) an amino acid residue at a position corresponding to position 419 is substituted with any of alanine and phenylalanine; and
(xii) an amino acid residue at a position corresponding to position 420 is substituted with any of alanine and phenylalanine.

In a still more preferred embodiment, in the recombinant polypeptide according to the present invention, in the amino acid sequence A, at least one of the following based on the amino acid sequence set forth in SEQ ID NO: 1 is satisfied:
(iv) an amino acid residue at a position corresponding to position 245 is substituted with any of arginine, serine, and tryptophan;
(v) an amino acid residue at a position corresponding to position 250 is substituted with leucine; and
(vii) an amino acid residue at a position corresponding to position 405 is substituted with any of lysine, histidine, serine, glutamine, tyrosine, and tryptophan.

The mutation in these amino acid sequences may be one or a combination of two or more of the substitutions.

When the mutation in the amino acid sequence is a combination of two or more, examples of preferred combinations of mutation-introducing positions include, but are not limited to, the following positions based on the amino acid sequence set forth in SEQ ID NO: 1 in the amino acid sequence A:
· a position corresponding to position 245 and a position corresponding to position 250;
· a position corresponding to position 245 and a position corresponding to position 405;
· a position corresponding to position 250 and a position corresponding to position 405; and
· a position corresponding to position 405 and a position corresponding to position 418.

Examples of a preferred combination of a mutation-introducing position and a mutation amino acid based on the amino acid sequence set forth in SEQ ID NO: 1 in the amino acid sequence A include, but are not limited to:
· an amino acid residue at a position corresponding to 245 position is arginine and an amino acid at a position corresponding to 250 position is leucine;
· an amino acid residue at a position corresponding to 245 position is arginine and an amino acid at a position corresponding to 405 position is tyrosine;
· an amino acid residue at a position corresponding to 245 position is tryptophan and an amino acid at a position corresponding to 405 position is tyrosine;
· an amino acid residue at a position corresponding to 245 position is arginine and an amino acid at a position corresponding to 405 position is histidine;
· an amino acid residue at a position corresponding to 250 position is leucine and an amino acid at a position corresponding to 405 position is tyrosine; and
· an amino acid residue at a position corresponding to 418 position is alanine and an amino acid at a position corresponding to 405 position is phenylalanine.

The substrate-binding site can be estimated, for example, by existing protein crystal structure information (Protein Data Bank, http://www.rcsb.org/) and enzyme-substrate-binding structure prediction using a computational scientific method.

When binding structure prediction is performed, for example, a crude model based on a crystal structure of an enzyme having a similar amino acid sequence can be acquired using protein three-dimensional structure prediction software represented by Swiss-model software (https://swissmodel.expasy.org/). If necessary, a model of a dynamic stabilization state can be acquired by subjecting the present model to molecular dynamics calculation.

It is possible to estimate the substrate-binding site by superimposing the model obtained by the method described above on the predicted existing enzyme-substrate-binding structure. The superposition of protein molecules can be carried out, for example, using the molecular graphic tool Pymol (https://pymol.org/2/) software.

Alternatively, a target substrate can be fitted to the model described above and subjected to molecular dynamics calculation to obtain a dynamic stabilization model of an enzyme-substrate-binding structure. Here, a transition state structure of the substrate in the target reaction is estimated by quantum chemical calculation and is used for enzyme-substrate-binding structure prediction, which is also useful for more accurate binding model prediction. In the resulting enzyme-substrate-binding structure prediction model, an amino acid present in the vicinity of a substrate molecule can be estimated by displaying an amino acid residue present within an arbitrary distance around the substrate molecule using, for example, Pymol software described above.

By preparing and screening a comprehensive mutant enzyme library in which point mutations are introduced into the enzyme amino acids (groups) estimated in this way, it is possible to specify a substrate-binding site that contributes to improvement of enzyme activity.

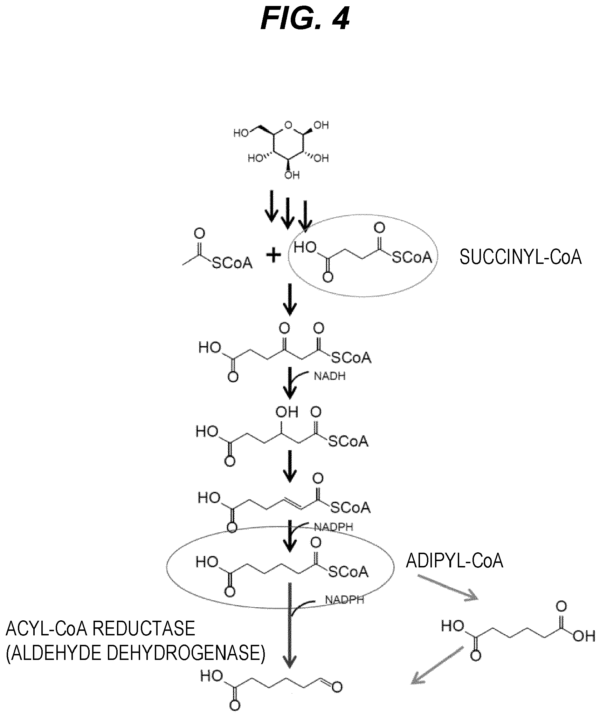

Regarding the recombinant polypeptide according to the present invention, the reducing activity R of converting CoA thioester of an acyl-CoA compound into an aldehyde group includes:
(c-1) a reducing activity R1 of converting adipyl-CoA into 5-formylpentanoic acid in one step; and
(c-2) a reducing activity R2 of converting succinyl-CoA into succinic semialdehyde (Fig. 4).

Regarding the reducing activity R1, the activity of "converting adipyl-CoA into 5-formylpentanoic acid in one step" refers to an activity of directly converting adipyl-CoA into 5-formylpentanoic acid, that is, of converting adipyl-CoA into 5-formylpentanoic acid, without generating an intermediate. In a case where adipyl-CoA is converted into 5-formylpentanoic acid, a two-step reaction is typically required in which CoA thioester of adipyl-CoA is converted into carboxyl groups to produce adipic acid, and then one of two carboxyl groups of adipic acid is further converted into an aldehyde group to produce 5-formylpentanoic acid (Fig. 4). Regarding the recombinant polypeptide according to the present invention, the reducing activity R1 directly converts adipyl-CoA into 5-formylpentanoic acid and does not produce adipic acid as an intermediate.

In a preferred embodiment, the reducing activity R1 is improved compared to the reducing activity R1' of a wild-type acyl-CoA compound reductase (for example, a polypeptide having an amino acid sequence set forth in SEQ ID NO: 1) that converts adipyl-CoA into 5-formylpentanoic acid in one step. The reducing activity R1 is improved, for example, by 1.1 times or more, and preferably 1.3 times or more, as compared with the reducing activity R1' of the wild-type acyl-CoA compound reductase.

Regarding the reducing activity R2 of converting succinyl-CoA into succinic semialdehyde, the reducing activity R2 is lower than the reducing activity R2' of the wild-type acyl-CoA compound reductase (for example, a polypeptide having an amino acid sequence set forth in SEQ ID NO: 1) that converts succinyl-CoA into succinic semialdehyde. The reducing activity R2 is, for example, 5/6 or less, preferably 2/3 or less, more preferably 1/2 or less, and most preferably 1/5 or less, of the reducing activity R2' of the wild-type acyl-CoA compound reductase.

The wild-type acyl-CoA compound reductase (for example, a polypeptide having an amino acid sequence set forth in SEQ ID NO: 1) has high reactivity to a succinyl-CoA substrate, which is an important intermediate of an adipyl-CoA route, and low reactivity to adipyl-CoA, which is a target substrate, and cannot produce a sufficient amount of formylpentanoic acid. On the other hand, in the recombinant polypeptide according to the present invention, as a specific amino acid mutation is introduced, the reducing activity R2 of converting succinyl-CoA into succinic semialdehyde is lowered compared to the wild-type acyl-CoA compound reductase, such that succinyl-CoA that is converted into succinic semialdehyde can be reduced, the amount of adipyl-CoA produced can be increased, and thus, formylpentanoic acid can be produced from adipyl-CoA.

Here, the reducing activity R (including R1, R1', R2, and R2') can be measured and evaluated by a method known in the art. For example, in the acyl-CoA reduction reaction according to the present application, nicotinamide adenine dinucleotide phosphate (NADPH) is consumed as a coenzyme in an amount of 1 equivalent per molecule of the substrate. Since NADPH is absorbed at 340 nm, a reducing activity can be evaluated by measuring a change in absorbance at 340 nm of an aqueous solution obtained by mixing an enzyme, a substrate, and a coenzyme.

The second aspect of the present invention is a DNA encoding the recombinant polypeptide. Specifically, it is a DNA encoding the amino acid sequence A. An example of the DNA encoding the recombinant polypeptide is the polynucleotide set forth in SEQ ID NO: 2.

Another aspect of the present invention is a recombinant microorganism into which the DNA encoding the recombinant polypeptide (for example, the polynucleotide set forth in SEQ ID NO: 2) has been introduced. Such a recombinant microorganism can be obtained by preparing a recombinant DNA by linking a DNA having a polynucleotide sequence encoding a recombinant polypeptide to a vector DNA, and transforming a bacterial strain of a host microorganism using the recombinant DNA. The recombinant microorganism into which the DNA encoding the recombinant polypeptide according to the present invention has been introduced has an adipyl-CoA production ability. The recombinant microorganism according to the present invention "having the adipyl-CoA production ability" means that the microorganism has a production pathway of adipyl-CoA. In the present specification, regarding a compound, the microorganism "having a production pathway" means that the microorganism can express a sufficient amount of an enzyme to allow each reaction step of the production pathway of the compound to proceed, and can biosynthesize the compound. The recombinant microorganism of the present invention may use a host microorganism having an adipyl-CoA production ability intrinsically, or may be modified so that a host microorganism originally not having an adipyl-CoA production ability intrinsically has an adipyl-CoA production ability.

As the host microorganism, various microorganisms that can be used in a fermentation process are available, and are selected from, for example, bacteria, yeast, and fungi. The host microorganism is selected from, for example, microorganisms of the genera *Escherichia, Bacillus, Corynebacterium, Klebsiella, Clostridium, Gluconobacter, Zymomonas, Lactobacillus, Lactococcus, Streptococcus, Pseudomonas,* and *Streptomyces.* The host microorganism is preferably *Escherichia coli* from the viewpoint that genetic recombination is easy.

The DNA of the recombinant polypeptide according to the present invention may be introduced into a host microorganism using a vector capable of autonomously replicating in the host microorganism, or the DNA of the recombinant polypeptide according to the present invention may be inserted into a chromosome and replicated. The vector is selected, for example, from the group consisting of a plasmid, a phage, a transposon, an IS element, a phasmid, a cosmid, and a linear and circular DNA. The DNA of the recombinant polypeptide is incorporated into a vector and introduced into a host microorganism. The vector is preferably a plasmid or phage.

In a case where the host microorganism is *Escherichia coli,* a suitable plasmid is, for example, pLG338, pACYC184, pBR322, pUC18, pUC19, pHSG298, pHSG398, pKC30, pRep4, pHS1, pKK223-3, pDHE19.2, pHS2, pPLc236, pMBL24, pLG200, pUR290, pIN-III113-B1, λgt11, and pBdCI. When the host microorganism is a bacillus such as the genus *Bacillus,* a preferred plasmid is, for example, pUB 110, pC 194, and pBD 214. Other usable plasmids are described in "Gene Cloning and DNA analysis 6th edition", Wiley-Blackwell 2016.

In the present invention, a vector can be prepared, for example, by operably linking a promoter (control region) upstream and a terminator downstream of a polynucleotide sequence encoding a gene, respectively, and in some cases, operably linking a gene marker and/or another control sequence.

In the preparation of the recombinant microorganism, an expression mechanism suitable for enhancing the expression of the recombinant polypeptide, for example, a promoter and a terminator can be selected and used. The promoter is defined as a DNA sequence that allows RNA polymerase to bind to DNA to initiate RNA synthesis, regardless of whether the promoter is a constitutive expression promoter or an inductive expression promoter. A strong promoter is a promoter that initiates mRNA synthesis at a high frequency, and is also suitably used in the present invention. In *Escherichia coli,* a major operator and promoter region of a lac system, a trp system, a tac or trc system, or λ-phage, a control region for fd coat protein, a promoter for a glycolytic enzyme (for example, 3-phosphoglycerate kinase or glyceraldehyde-3-phosphate dehydrogenase), glutamate decarboxylase A, or serine hydroxymethyltransferase, and the like can be used. Examples of the terminator include an rrnBT1T2 terminator and a lac terminator.

Other regulatory elements that can be used in addition to promoter and terminator sequences are, for example, a selectable marker, an amplification signals, and a replication point. A regulatory element is described in, for example, "Gene Expression Technology: Methods in Enzymology 185", Academic Press (1990).

The host microorganism used in the present invention may be a microorganism in which a gene encoding an alcohol dehydrogenase inherent in the microorganism (hereinafter, also referred to as an "alcohol dehydrogenase gene") is disrupted. By using such a host microorganism, contamination of an alcohol dehydrogenase unique to the host microorganism can be avoided, and progress of an unintended reaction can be suppressed.

Still another aspect of the present invention relates to a culture of the recombinant microorganism described above and/or an extract of the culture.

A desired target compound can be produced using the recombinant polypeptide according to the present invention or a bacterial cell of a recombinant microorganism expressing the recombinant polypeptide. For example, in a case where the target compound is an alcohol, the alcohol can be produced by adding an aldehyde reductase or co-expressing an aldehyde reductase in a host microorganism. For example, in a case where the target compound is an aldehyde, the aldehyde can be produced by adding a carboxylate reductase or co-expressing a carboxylic acid in a host microorganism. In a case where the target compound is an amino compound, the amino compound can be produced by adding an aminotransferase or co-expressing an aminotransferase in a host microorganism. That is, there is also provided a method for culturing a recombinant microorganism obtained by introducing a DNA encoding the recombinant polypeptide according to the present invention into a host microorganism, and producing a target compound using the culture.

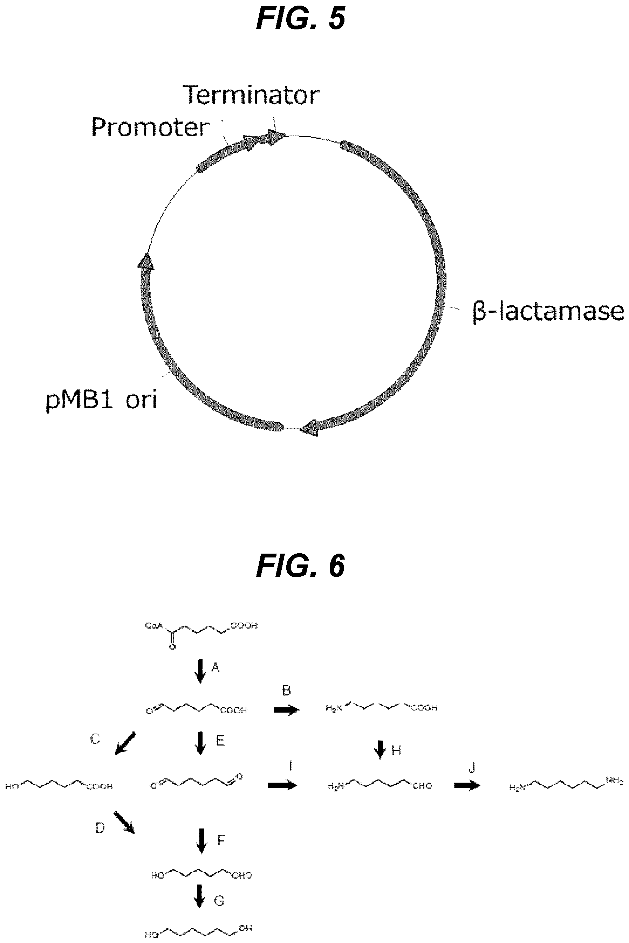

Therefore, still another aspect of the present invention relates to a production method of a target compound using the recombinant polypeptide or recombinant microorganism described above. Specifically, the production method includes mixing a recombinant polypeptide or a culture of a recombinant microorganism and/or an extract of the culture with a substrate compound to obtain a mixed solution. A reaction time for reacting the culture and/or the extract of the culture with the substrate compound in the mixed solution is a time during which the target product can be produced. The reaction time is, for example, 24 hours to 7 days in a case where the microorganism expressing the recombinant polypeptide according to the present invention is cultured, that is, in a case where the culture of the recombinant microorganism is mixed with the substrate compound. In addition, in a case where the recombinant polypeptide according to the present invention is isolated from the culture, that is, in a case where the recombinant polypeptide is mixed with the substrate compound as an extract extracted from the culture of the recombinant microorganism, the reaction time is, for example, 15 minutes to 48 hours. Here, the target compound is, for example, 5-formylpentanoic acid, 6-aminocaproic acid, 1,6-diaminohexane, 6-hydroxyhexanoic acid, hexanedial, 6-aminohexanal, 6-hydroxyhexanal, or 1,6-hexanediol (see Fig. 6).

In an embodiment, the production method according to the present invention includes converting adipyl-CoA into 5-formylpentanoic acid in the presence of the recombinant polypeptide described above or a culture of the recombinant microorganism described above and/or an extract of the culture, and the target compound is selected from the group consisting of 5-formylpentanoic acid, 6-aminocaproic acid, 1,6-diaminohexane, 6-hydroxyhexanoic acid, hexanedial, 6-aminohexanal, 6-hydroxyhexanal, and 1,6-hexanediol. The target compound is preferably 6-aminocaproic acid, 1,6-diaminohexane, 6-hydroxyhexanoic acid, or 1,6-hexanediol.

In a case where the target compound is 6-aminocaproic acid, the production method further includes converting 5-formylpentanoic acid into 6-aminocaproic acid (see reaction B in Fig. 6). In this case, the production method according to the present invention may include adding an aminotransferase or co-expressing an aminotransferase in a host microorganism, in addition to a modified aldehyde dehydrogenase (the recombinant polypeptide of the present invention).

In a case where the target compound is 6-hydroxyhexanoic acid, the production method further includes converting 5-formylpentanoic acid into 6-hydroxyhexanoic acid (see reaction C in Fig. 6). In this case, the production method according to the present invention may include adding an alcohol dehydrogenase or co-expressing an aldehyde reductase in a host microorganism, in addition to a modified aldehyde dehydrogenase (the recombinant polypeptide of the present invention).

In a case where the target compound is 1,6-hexanediol, the production method further includes i) converting the 5-formylpentanoic acid into 6-hydroxyhexanoic acid (reaction C in Fig. 6), converting the 6-hydroxyhexanoic acid into 6-hydroxyhexanal (reaction D in Fig. 6), and converting the 6-hydroxyhexanal into 1,6-hexanediol (reaction G in Fig. 6). In this case, the production method according to the present invention may include adding an alcohol dehydrogenase and a carboxylate reductase or co-expressing these enzymes in a host microorganism, in addition to a modified aldehyde dehydrogenase (the recombinant polypeptide of the present invention).

In a case where the target compound is 1,6-hexanediol, the production method further includes ii) converting the 5-formylpentanoic acid into hexanedial (reaction E in Fig. 6), converting the hexanedial into 6-hydroxyhexanal (reaction F in Fig. 6), and converting the 6-hydroxyhexanal into 1,6-hexanediol (reaction G in Fig. 6). In this case, the production method according to the present invention may include adding an aldehyde reductase and a carboxylate reductase or co-expressing these enzymes in a host microorganism, in addition to a modified aldehyde dehydrogenase (the recombinant polypeptide of the present invention).

In a case where the target compound is 1,6-diaminohexane, the production method further includes i) converting the 5-formylpentanoic acid into 6-aminocaproic acid (reaction B in Fig. 6), converting the 6-aminocaproic acid into 6-aminohexanal (reaction H in Fig. 6), and converting the 6-aminohexanal into 1,6-diaminohexane (reaction J in Fig. 6). In this case, the production method according to the present invention may include adding an aminotransferase and a carboxylate reductase or co-expressing these enzymes in a host microorganism, in addition to a modified aldehyde dehydrogenase (the recombinant polypeptide of the present invention).

In a case where the target compound is 1,6-diaminohexane, the production method further includes ii) converting the 5-formylpentanoic acid into hexanedial (reaction E in Fig. 6), converting the hexanedial into 6-aminohexanal (reaction I in Fig. 6), and converting the 6-aminohexanal into 1,6-diaminohexane (reaction J in Fig. 6). In this case, the production method according to the present invention includes adding an aminotransferase and a carboxylate reductase or co-expressing these enzymes in a host microorganism, in addition to a modified aldehyde dehydrogenase (the recombinant polypeptide of the present invention).

The reaction of converting the 5-formylpentanoic acid into 6-aminocaproic acid (reaction B in Fig. 6), the reaction of converting the hexanedial into 6-aminohexanal (reaction I in Fig. 6), and the reaction of converting the 6-aminohexanal into 1,6-diaminohexane (reaction J in Fig. 6) are catalyzed by an aminotransferase, and the aminotransferase is, for example, selected from the group consisting of:
· 4-aminobutanoate-2-oxoglutarate transaminase (EC 2.6.1.19);
· putrescine-2-oxoglutarate transaminase (EC 2.6.1.82);
· 4-aminobutanoate-pyruvate transaminase (EC 2.6.1.96); and
· putrescine-pyruvate transaminase (EC 2.6.1.113). These aminotransferases are derived, for example, from microorganisms selected from the group consisting of the genus *Vibrio* and the genus *Escherichia.*

The reaction of converting the 6-hydroxyhexanoic acid into 6-hydroxyhexanal (reaction D in Fig. 6), the reaction of converting the 5-formylpentanoic acid into hexanedial (reaction E in Fig. 6), and the reaction of converting the 6-aminocaproic acid into 6-aminohexanal (reaction H in Fig. 6) are catalyzed by an enzyme belonging to a carboxylate reductase (EC 1.2.1.30). These carboxylate reductases are derived, for example, from microorganisms selected from the group consisting of the genus *Mycobacterium* and the genus *Nocardia.*

The reaction of converting the 5-formylpentanoic acid into 6-hydroxyhexanoic acid (reaction C in Fig. 6), the reaction of converting the hexanedial into 6-hydroxyhexanal (reaction F in Fig. 6), and the reaction of converting the 6-hydroxyhexanal into 1,6-hexanediol (reaction G in Fig. 6) are catalyzed by an alcohol dehydrogenase, and the alcohol dehydrogenase is selected, for example, from the group consisting of:
· alcohol dehydrogenase (EC 1.1.1.1); and
· alcohol dehydrogenase (EC 1.1.1.2). The alcohol dehydrogenase is derived, for example, from a microorganism selected from the group consisting of the genus *Escherichia* and the genus *Bacillus.*

Still another aspect of the present invention relates to a recombinant microorganism having a pathway for producing at least one selected from the group consisting of 5-formylpentanoic acid, 6-aminocaproic acid, 1,6-diaminohexane, 6-hydroxyhexanoic acid, hexanedial, 6-aminohexanal, 6-hydroxyhexanal, and 1,6-hexanediol, and the recombinant microorganism has an exogenous nucleic acid sequence encoding:
· an enzyme having an amino acid sequence set forth in SEQ ID NO: 1; or
· an enzyme having an amino acid sequence having a sequence identity of 60% or higher, 65% or higher, 70% or higher, 75% or higher, 80% or higher, 85% or higher, 88% or higher, 90% or higher, 93% or higher, 95% or higher, 97% or higher, 98% or higher, or 99% or higher with the amino acid sequence set forth in SEQ ID NO: 1 and having a reducing activity R of converting CoA thioester of an acyl-CoA compound into an aldehyde group.

The production method according to the present invention may further include culturing the recombinant microorganism described above, accumulating any one or more of the target compounds in a culture solution, and isolating and/or purifying the target compound.

The conditions for allowing the acyl-CoA reduction reaction to proceed in the presence of the modified aldehyde dehydrogenase according to the present invention may be any conditions under which a target product is produced, and can be set by adjusting the composition, pH, reaction temperature, reaction time, and the like of the reaction solution by a method usually performed by those skilled in the art. Examples of the pH of the reaction solution include a buffer solution having a pH of 7 to 9, and examples of more preferred conditions include a buffer solution containing HEPES-KOH having a pH of 8 to 9. The reaction temperature is usually 20 to 40°C and more preferably 30 to 37°C. The reaction time may be any time duration during which the target product can be produced, and is, for example, 24 hours to 7 days in a case where the culture of the recombinant microorganism according to the present invention is mixed with the substrate compound, and is, for example, 15 minutes to 48 hours in a case where the recombinant polypeptide according to the present invention is extracted from the culture of the recombinant microorganism and mixed with the substrate compound.

The culture of the recombinant microorganism and/or the extract of the culture may be prepared as an enzyme-containing product containing the recombinant polypeptide according to the present invention, for example, an enzyme solution. In a method for preparing the enzyme solution, a host microorganism expressing the enzyme according to the present invention is subjected to, for example, sonication disruption, bead mill disruption, or lysis using lysozyme, and a centrifugal supernatant thereof can be used.

A medium composition, culture conditions, and culture time for culturing the recombinant microorganism according to the present invention can be appropriately selected by a method usually performed by those skilled in the art. The culture temperature is typically in a range of 20 to 40°C and preferably 30 to 37°C.

The medium may be a natural, semi-synthetic, or synthetic medium containing one or more carbon sources, nitrogen sources, inorganic salts, vitamins, or a trace amount of an optional element or vitamin. The medium to be used should adequately meet the nutritional requirements of the transformants to be cultured. When the host microorganism is aerobic, shaking should be performed to ensure a suitable oxygen concentration during fermentation. These culture conditions can be easily set by those skilled in the art.

In addition, the medium may contain a corresponding antibiotic in a case where a transformant expresses a useful additional trait, for example, in a case where a transformant contains a marker resistant to an antibiotic. By adding an antibiotic, a plasmid is stably retained. Examples of the antibiotic include, but are not limited to, ampicillin, kanamycin, chloramphenicol, tetracycline, erythromycin, streptomycin, and spectinomycin.

According to the present invention, it is possible to provide a recombinant polypeptide having an excellent acyl-CoA compound reducing activity and a production method of an aliphatic compound using the recombinant polypeptide. Specifically, a specific mutation is introduced into the wild-type acyl-CoA compound reductase, such that the enzyme activity for the substrate can be adjusted, and a modified enzyme having an excellent enzyme activity as compared with the wild-type acyl-CoA compound reductase can be obtained. Specifically, the activity for succinyl-CoA is reduced, such that the amount of adipyl-CoA produced is increased, and 5-formylpentanoic acid can be obtained from adipyl-CoA. Therefore, the target compound can be efficiently produced by using the modified enzyme according to the present invention. In addition, various target compounds, particularly, an aliphatic compound, can be obtained from 5-formylpentanoic acid by performing conversion using an additional enzyme according to a desired target compound. Furthermore, the modified enzyme according to the present invention is expected to be usable for the production of a target compound on an industrial scale because the enzyme activity for a substrate is regulated and a target compound can be efficiently produced.

Those skilled in the art given the above description can sufficiently implement the present invention. Hereinafter, the working examples are given for the purpose of further explanation, and the present invention is not limited to the working examples.

### Examples

All the PCRs shown in the present working examples were performed using PrimeSTAR Max DNA Polymerase (Takara Bio Inc.). As for a transformation of *Escherichia coli,* the calcium chloride method (see Genetic Engineering Experiment Note, first volume, YODOSHA CO., LTD., written by Takaaki Tamura) was used. In the construction of the plasmid, an LB medium was used, and a necessary antibiotic was added thereto. As a reagent used in the present working examples, a reagent manufactured by FUJIFILM Wako Pure Chemical Corporation was used unless otherwise specified. As a thermal cycler, C1000Touch touch thermal cycler manufactured by Bio-Rad Laboratories, Inc. was used.

The composition of the medium used in each example is as follows.

### (LB Medium)

20 g/L of an LB medium and Lenox (manufactured by Difco Laboratories, Inc.) were subjected to steam sterilization at 120°C for 20 minutes. If necessary, a final concentration 100 mg/L of ampicillin and 1.5% Bacto agar (manufactured by Difco Laboratories, Inc.) were added.

### (SOC Medium)

First, 1 L of a solution of 20 g/L bacto toryptone (manufactured by Difco Laboratories, Inc.), 5 g/L bacto yeast extract (manufactured by Difco Laboratories, Inc.), 1 M NaCl, and 1 M KCl was prepared, steam sterilization was performed at 120°C for 20 minutes, and the solution was cooled to room temperature. Next, 10 ml of each of 1 M MgSO₄, 1 M MgCl₂, and 2 M glucose was sterilized by filtration, and added to the mixed solution described above.

### <1> Estimation of Enzyme Activity Center

In preparing a modified enzyme, amino acid residues to be considered to form an active center were estimated according to the following method. The protein crystal structure of the polypeptide of SEQ ID NO: 1 as a template has not been reported. Therefore, first, a crude model of the three-dimensional structure of the polypeptide of SEQ ID NO: 1 was prepared using Swiss-model (https://swissmodel.expasy.org/, Swiss Institute of Bioinformatics) software. At this time, PDB entry registered as "5j78" was used as a template. Next, pymol software was used to determine the amino acid residues in the space of 5 angstroms around propionyl-CoA in the model of "5jfn" in PDB, and the model of "5jfn" was superimposed with the model of "4c3s" to estimate the amino acids corresponding to the above in "4c3s". Furthermore, the model of SEQ ID NO: 1 and the model of "4c3s" were superimposed, and amino acid residues overlapping the amino acid residue in "4c3s" determined by superimposition with the model of "5jfn" above in the model of SEQ ID NO: 1 were extracted.

### <2> Construction of Plasmid

### (Construction of Vector)

A vector for gene expression was prepared as follows. First, a promoter sequence was inserted using BglII and EcoRV sites of the multiple cloning site of the plasmid pNFP-A51 (deposited on October 25, 2011 as FERM P-22182, Independent Administrative Institution, National Institute of Technology and Evaluation, International Patent Organism Depositary (IPOD) (address: Central 6, 1-1-1 Higashi, Tsukuba, Ibaraki, Japan), International Deposition Accession No. FERM BP-11515), and a terminator sequence was inserted using XbaI and HindIII sites, thereby constructing pSK000. At this time, three base pairs upstream of the EcoRV restriction enzyme recognition sequence were added to the promoter sequence terminal so that the EcoRV restriction enzyme site remained between the promoter and terminator sequences, and the EcoRV restriction enzyme site was used at the time of enzyme gene cloning described below. The promoter sequence and the terminator sequence are shown in Table 1. The pSK000 plasmid structure is illustrated in Fig. 5. The restriction enzymes used were manufactured by Takara Bio Inc.

**[Table 1]**

| | Base sequence | SEQ ID NO: |
|---|---|---|
| Promoter | CTCAAGCCCAAAGGAAGAGTGAGGCGAGTCAG TCGCGTAATGCTTAGGCACAGGATTGATTTGTC GCAATGATTGACACGATTCCGCTTGACGCTGCG TAAGGTTTTTGTAATTTTACAGGCAACCTTTTAT TCACTAACAAATAGCTGGTGGAA | 3 |
| Terminator | AAAAAAAAAAGGTGCCGTGGAAACACGGCAC CTTTTTTTTTTCTTTA | 4 |

### (Preparation of Enzyme Expression Plasmid)

Polynucleotides encoding an aminotransferase were synthesized using an artificial gene synthesis service of Eurofins Genomics K.K. The aldehyde dehydrogenase gene was amplified using primers 1 and 2. Gene sequences encoding an aminotransferase were obtained from NCBI Accession No. DQ904035 and amplified using primers 3 and 4. Gene sequences encoding an aldehyde reductase were obtained from NCBI Accession No. AP009048 and amplified using primers 5 and 6. The respective primer sequences are as shown in Table 2. Each gene was amplified under the reaction conditions of 98°C (10 sec), 55°C (5 sec), 72°C (10 sec), and 30 cycles.

**[Table 2]**

| Primer No. | Base sequence | SEQ ID NO: |
|---|---|---|
| 1 | ATGGAAAAAGCAGTCGAGAAC | 5 |
| 2 | TTAGCCCCATAATTCTTCGTC | 6 |
| 3 | ATGATACGCGAGCCTCCGGA | 7 |
| 4 | TTACGCTTCTTCGACACTTAC | 8 |
| 5 | ATGTCGATGATAAAAAGCTATGCC | 9 |
| 6 | TCAAAAATCGGCTTTCAACACC | 10 |

The resulting DNA fragment was phosphorylated with Mighty Cloning Reagent Set (manufactured by Takara Bio Inc.) and used as a DNA fragment to be cloned. Separately, the pSK000 plasmid was treated with an EcoRV restriction enzyme and then was treated with alkaline phosphatase (BAP, manufactured by Takara Bio Inc.). The DNA fragment and the vector fragment were ligated using Mighty Cloning Reagent Set (manufactured by Takara Bio Inc.) (16°C, overnight). An *Escherichia coli* JM109 strain was transformed with 1 µL of a ligation reaction solution. The plasmid was extracted from the appeared colonies and subjected to sequence analysis to prepare a plasmid into which a gene was inserted so that the enzyme protein was expressed.

### (Preparation of Point Mutation Introduced-Enzyme Expression Plasmid)

Point mutation introduction into the aldehyde dehydrogenase gene sequence was performed using the principle of inversePCR. The primers used for constructing the respective mutant expression plasmids are as shown in Tables 3-1 and 3-2. In Table 3-2, primers without notation were forward primers, and for the reverse primers, common reverse primers were used for the amino acid mutation at the position specified in the table regardless of the amino acid species after the mutation. The gene was amplified under the reaction conditions of 98°C (10 sec), 55°C (5 sec), 72°C (10 sec), and 30 cycles. The resulting DNA fragments were purified and phosphorylated and subjected to self-ligation at 16°C for 20 minutes. The *Escherichia coli* JM109 strain was transformed using 1 µL of the reaction solution. Plasmids were extracted from the appeared colonies and subjected to sequence analysis to obtain plasmids into which target mutations were correctly introduced.

In the second mutation introduction, a plasmid was prepared by repeating the above steps using the plasmid into which the point mutation was introduced as a template.

**[Table 3-1]**

| Mutation site | Introduced amino acid | Forward primer | SEQ ID NO: | Reverse primer | SEQ ID NO: |
|---|---|---|---|---|---|
| A74 | F | tttaaaaacaaaagcaaagccaaa gtcatctacaa | 11 | aactttgtcttcatagactccca tacccgt | 12 |
| | R | cgtaaaaacaaaagcaaagccaa agtcatc | 13 | aactttgtcttcatagactccca tacccgt | 14 |
| K75 | A | gctaacaaagcaaagccaaagtc atctacaa | 15 | agcaactttgtcttcatagactc ccatacc | 16 |
| | F | tttaacaaaagcaaagccaaagtc atctacaa | 17 | agcaactttgtcttcatagactc ccatacc | 18 |
| S78 | A | gctaaagccaaagtcatctacaaca atctgaa | 19 | tttgtttttagcaactttgtcttca tagactcc | 20 |
| | F | tttaaagccaaagtcatctacaaca atctgaagga | 21 | ttgtttttagcaactttgtcttca tagactcc | 22 |
| K79 | A | gctgccaaagtcatctacaacaatc tgaagg | 23 | gcttttgtttttagcaactttgtct tcatagac | 24 |
| | F | tttgccaaagtcatctacaacaatct gaagg | 25 | gcttttgtttttagcaactttgtct tcatagac | 26 |
| F245 | A | gctgataatgggatcattgctctgg c | 27 | aatgcgcccggcaataatcttt gg | 28 |
| | R | cgtgataatgggatcatttgctctgg c | 29 | aatgcgcccggcaataatcttt gg | 30 |
| S410 | A | gcttttttcaatggcttagcgccc | 31 | gccacctgcagacgtcgc | 32 |
| | F | ttttttttcaatggcttagcgcccacg | 33 | gccacctgcagacgtcgc | 34 |
| F411 | A | gctttcaatggcttgacgcccac | 35 | gctgccacctgcagacgtcgc | 36 |
| | R | cgtttcaatggcttagcgcccac | 37 | gctgccacctgcagacgtcgc | 38 |
| I250 | F | ttctgctctggcgaacagagtgtcat tg | 39 | gatcccattatcaaaaatgcgc ccggc | 40 |
| | A | gcgtgctctggcgaacagagtgtc attg | 41 | gatcccattatcaaaaatgcgc ccggc | 42 |
| C251 | F | ttttctggcgaacagagtgtcattgt ggc | 43 | aatgatcccattatcaaaaatg cgcccggc | 44 |
| S252 | F | [?] tttggcgaacagagtgtcattgtgg cgg | 45 | [?] gcaaatgatcccattatcaaaa atgcgcccg | 46 |
| | A | gcgggcgaacagagtgtcattgtg gcgg | 47 | atgcgcccg gcaaatgatcccattatcaaaa atgcgcccg | 48 |
| A404 | F | tttacgtctgcaggtggcagctttttc a | 49 | acagcactgattgatcacaaa acgagacacc | 50 |
| T405 | F | ttttctgcaggtggcagctttttcaat ggcttag | 51 | cgcacagcactgattgatcac aaaacgag | 52 |
| | A | gcgtctgcaggtggcagctttttcaa tggcttag | 53 | cgcacagcactgattgatcac aaaacgag | 54 |
| S406 | F | ttgcaggtggcagctttttcaatgg cttagc | 55 | cgtcgcacagcactgattgatg acaaaac | 56 |
| | A | gcggcaggtggcagcttttcaatg gcttagc | 57 | cgtcgcacagcactgattgatc acaaaac | S8 |
| A407 | F | tttggtggcagctttttcaatggctta gcgc | 59 | agacgtcgcacagcactgatt gatcac | 60 |
| G408 | F | tttggcagcttttcaatggcttagcg ccc | 61 | tgcagacgtcgcacagcactg attgatc | 62 |
| G409 | F | tttagctttttcaatggcttagcgccc acg | 63 | acctgcagacgtcgcacagca ctg | 64 |
| 1418 | F | tttaataccttgggctgtggctcatg gg | 65 | gggcgctaagccattgaaaaa gctgcc | 66 |
| | A | gcgaataccttgggctgtggctcat ggg | 67 | gggcgctaagccattgaaaaa gctgcc | 68 |
| N419 | F | tttaccttgggctgtggctcatggg | 69 | cgtgggcgctaagccattgaa aaagc | 70 |
| | A | gcgaccttgggctgtggctcatggg | 71 | cgtgggcgctaagccattgaa aaagc | 72 |
| T420 | F | ttcttgggctgtggctcatggggc | 73 | attcgtgggcgctaagccattg aaaaag | 74 |
| | A | gcgttgggctgtggctcatggc | 75 | attcgtgggcgctaagccattg aaaaag | 76 |

**[Table 3-2]**

| SEQ ID NO: | Primer name | Base sequence |
|---|---|---|
| 122 | I250K | atcaaatgctctggcgaacagagtgtca |
| 123 | I250R | atccgctgctctggcgaacagagtgtca |
| 124 | I250H | atccactgctctggcgaacagagtgtca |
| 125 | I250Q | atcacctgctctggcgaacagagtgtca |
| 126 | I250T | atccagtgctctggcgaacagagtgtca |
| 127 | I250N | atcaactgctctggcgaacagagtgtca |
| 128 | I250G | atcggctgctctggcgaacagagtgtca |
| 129 | 1250L | atcgtgtgctctggcgaacagagtgtca |
| 130 | I250V | atcctgtgctctggcgaacagagtgtca |
| 131 | I250W | atctggtgctctggcgaacagagtgtca |
| 132 | I250P | atcccgtgctctggcgaacagagtgtca |
| 133 | 1250 Reverse primer | cccattatcaaaaatgcgcccggcaataatcttt |
| 134 | F245R | attcgcgataatgggatcatttgctctggcgaa |
| 135 | F245E | attgaagataatgggatcatttgctctggcgaa |
| 136 | F245S | attagcgataatgggatcatttgctctggcgaa |
| 137 | F245N | attaacgataatgggatcatttgctctggcgaa |
| 138 | F245Q | attcaggataatgggatcatttgctctggcgaa |
| 139 | F245G | attggcgataatgggatcatttgctctggcgaa |
| 140 | F245L | attctggataatgggatcatttgctctggcgaa |
| 141 | F245W | atttgggataatgggatcatttgctctggcgaa |
| 142 | F245 Reverse primer | gcgcccggcaataatctttggcacc |
| 143 | T405K | gcgaaatctgcaggtggcagctttttcaatggct |
| 144 | T40SH | gcgcactctgcaggtggcagctttttcaatggct |
| 145 | T405E | gcggaatctgcaggtggcagctttttcaatggct |
| 146 | T405S | gcgagctctgcaggtggcagctttttcaatggct |
| 147 | T405N | gcgaactctgcaggtggcagctttttcaatggct |
| 148 | T405Q | gcgcagtctgcaggtggcagctttttcaatggct |
| 149 | T405V | gcggtgtctgcaggtggcagctttttcaatggct |
| 150 | T40SL | gcgctgtctgcaggtggcagctttttcaatggct |
| 151 | T405Y | gcgtattctgcaggtggcagctttttcaatggct |
| 152 | T405W | gcgtggtctgcaggtggcagctttttcaatggct |
| 153 | T405P | gcgccgtctgcaggtggcagctttttcaatggct |
| 154 | T405F | gcgttttctgcaggtggcagctttttcaatggct |
| 155 | T405 Reverse primer | acagcactgattgatcacaaaacgagacacctcaa |
| 156 | T418A | gcgaataccttgggctgtggctcatggg |
| 157 | T418 Reverse primer | gggcgctaagccattgaaaaagctgcc |

### <3> Evaluation of Activity of Point Mutant Enzymes for Various Substrates

### (Synthesis of Adipyl-CoA)

Adipyl-CoA was synthesized with reference to the literature (Manning et al. 2018 Biochemical and Biophysical Research Communications). On ice, 100 mg of a coenzyme A (hereinafter, referred to as "CoA") manufactured by Oriental Yeast Co., Ltd. was dissolved in 2.5 mL of 0.5 M KHCO₃, and 250 µL of 1 M HCl was added thereto. Separately, 16 mg of adipic anhydride was suspended in 2.5 mL of acetonitrile, a CoA aqueous solution was added while stirring with a magnetic stirrer, and the mixed solution was stirred at 4°C for 24 hours. The reaction solution was dispensed into 1.5 mL tubes by 500 µL, and concentrated for 2 hours using CVE-3110 centrifugal evaporator manufactured by Tokyo Rikakikai Co., Ltd. Thereafter, freezing was performed at -80°C for 3 hours, and then drying was performed in a vacuum dryer overnight. Separately, the amount of residual CoA in the synthetic product was quantified by a color reaction with 5,5'-dithio-bis-2-nitrobenzoic acid (DTNB) to determine the amount of adipyl-CoA produced. Reaction conditions; synthetic sample diluted with water 4 times or CoA preparation aqueous solution 15 µL, Tris-HCl (pH8.0) 30 µL, 10 mM DTNB 5 µL, and they were diluted with water so that the total amount is 300 µL. After mixing, the mixed solution was allowed to stand at normal temperature for 5 minutes, and the absorbance at 412 nm was measured.

Synthetic adipyl-CoA was purified with a column by the following procedure. Sep-Pak Vac 2 g/12 cc C18 (manufactured by Waters Corporation) column was conditioned with 10 mL of methanol. Next, 10 mL of a 0.1% TFA aqueous solution was allowed to pass through the column. Then, the lyophilized solid was dissolved in 1 mL of water and passed through the column. The column was washed 3 times with 10 mL of a 0.1% TFA aqueous solution. 2 mL of 0.1% TFA 50% acetonitrile solution was passed through the column twice to elute the target product. After sampling 10 uL for analysis, 500 uL of the remainder was dispensed into 1.5 mL tubes and concentrated for 2 hours using a centrifugal evaporator. Thereafter, freezing was performed at -80°C for 3 hours, and then drying was performed in a vacuum dryer overnight. The adipyl-CoA concentration of the purified product was determined by comparing the peak area with that of the synthetic product before purification using the LC analysis method shown in Table 4.

**[Table 4]**

| Analysis of adipyl-CoA | | | |
|---|---|---|---|
| Column | Shodex Asahipak GS-320 HQ (7.5 mm I.D. × 300 mm) | | |
| Column temperature | 30°C | | |
| Eluant | A | 205 mM | NaH₂PO₄ |
| | B | 205 mM | H₃PO₄ |
| | A:B | 300/7 | |
| Flow rate | 0.6 ml/min | | |
| Detector | UV 260 nm | | |
| Injection amount | 20 uL | | |

### (Evaluation of Activity of Mutation-Introduced Enzyme)

An *Escherichia coli* JM109 strain was transformed with a point mutanted enzyme expression plasmid. Similarly, for comparison, an *Escherichia coli* JM109 strain was transformed using either a wild-type enzyme expression plasmid or an empty plasmid containing no enzyme gene, and the transformed strain was cultured in an agar medium at 30°C for 1 day. The appeared colonies were inoculated into an LB medium (2 ml volume deep well plate, liquid volume 1 mL), and culture was performed at 37°C by shaking overnight.
30 uL thereof were dispensed into a microplate and diluted 10-fold, and an absorbance at 600 nm was measured using Tecan Infinite 200 microplate reader. 500 µL of the deep well plate culture solution was centrifuged with a floor-type plate centrifuge sorvall ST-8 manufactured by Thremo Scientific Inc. at 2,456 rpm for 10 minutes. The supernatant was pipetted off and discarded, and the pellets were lysed with 200 µL of B-PER. The lysis solution was centrifuged at 2,456 rpm for 10 minutes, and the supernatant was used as an enzyme solution. 40 µL of the enzyme solution was dispensed into the microplate, and the substrate solution preheated at 30°C was added (total amount: 200 µL, final concentration: 0.2 mM NADPH, 100 mM HEPES potassium buffer pH = 8.0). When adipyl-CoA was used as a substrate, 40 µL of the enzyme solution was used, and the substrate concentration was 1 mM. When succinyl-CoA was used as a substrate, the enzyme solution was diluted 10-fold and used at 20 µL so that a substrate concentration was 0.5 mM. The succinyl-CoA was purchased from Merck. An absorbance at 340 nm was serially measured for 30 minutes using Tecan Infinite 200 microplate reader. In addition, 5 µL of the assay solution after the reaction was sampled, and the total protein concentration was calculated on the basis of the Bradford method using a protein assay concentrated dye reagent manufactured by Bio-Rad Laboratories, Inc..

Separately, 5 µL of the enzyme solution was fractionated as a sample for SDS-PAGE, and mixed with 5 µL of a 2x laemmuli sample buffer (5% 2-mercapto-1,3-propanediol) manufactured by Bio-Rad Laboratories, Inc. The solution was heated at 100°C for 20 minutes, and then the heated solution was subjected to electrophoresis at 200 V for 30 minutes using Mini-PROTEAN TGX Gel (Any kD) manufactured by Bio-Rad Laboratories, Inc. and Mini-PROTEAN Tetra Cell SDS-PAGE electrophoresis tank. The gel after electrophoresis was dyed for 30 minutes with Coomassie stain solution manufactured by Bio-Safe, and decolorized with distilled water for 2 hours. The bleached gel was imaged with Gel DocEZ system manufactured by Bio-Rad Laboratories, Inc., and a proportion of the target enzyme band in the total protein bands of each lane was calculated using Image lab software. The total protein concentration was multiplied by the target enzyme band proportion to obtain the target enzyme concentration in the assay solution.

A consumption rate of NADPH was calculated from a change in absorbance at 340 nm. At this time, a value of the sample to which the disrupted supernatant of the empty plasmid-transformed strain was added was subtracted as a blank. An NADPH consumption rate per unit enzyme protein was calculated from the enzyme concentration described above. All values are mean values of two independent culture samples.

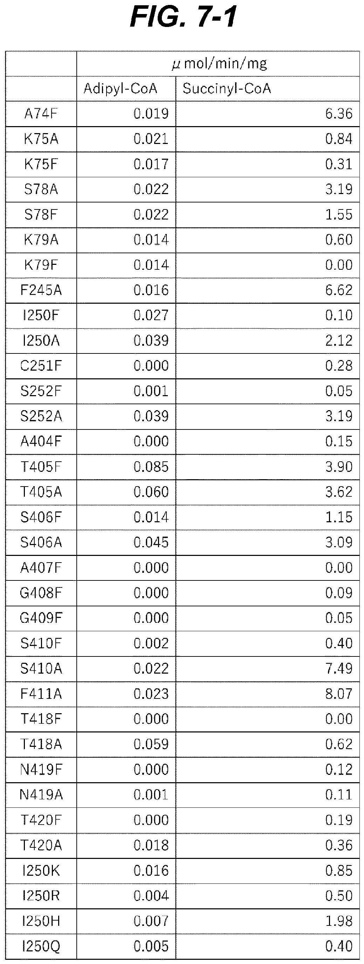

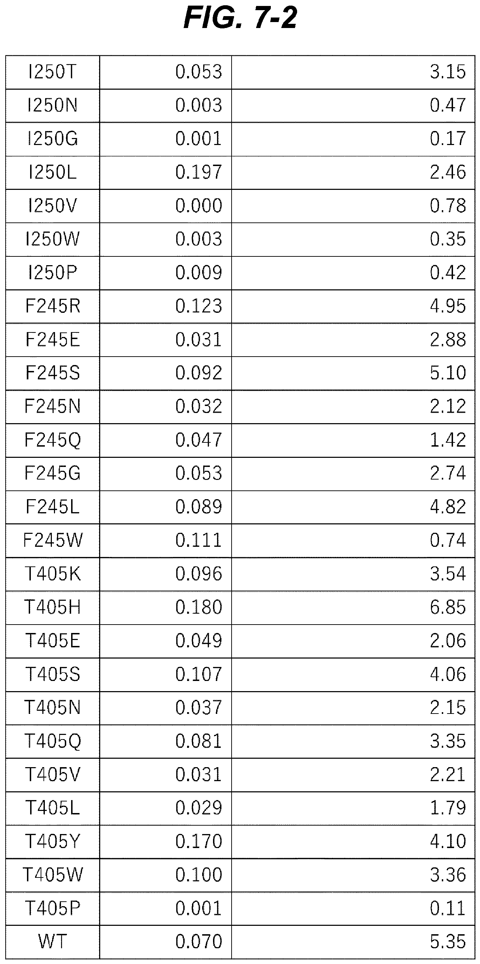

The activity of the mutant enzyme for each substrate is illustrated in Fig. 7. A plurality of mutant enzymes with a reduced succinyl-CoA activity and a plurality of mutant enzymes with an enhanced adipyl-CoA activity could be obtained.

### <4> Synthesis of Target Compound Using Mutant Enzyme

When an enzyme solution was prepared using *Escherichia coli,* an alcohol dehydrogenase ADH gene-disrupted strain was used as an *Escherichia coli* strain expressing an enzyme in order to avoid contamination with an alcohol dehydrogenase unique to *Escherichia coli.* The preparation method will be described below.

### (Preparation of ADH Gene Disruption Plasmid)

Disruption of an ADH gene was performed by a homologous recombination method using pHAK1 (as NITE P-02919, deposited with biotechnology division of National Institute of Technology and Evaluation (NITE), Patent Microorganisms Depositary (NPMD) (address: #122, 2-5-8, Kazusakamatari, Kisarazu-shi, Chiba, Japan) on March 18, 2019, International Accession No: NITE BP-02919). pHAK1 includes a temperature-sensitive mutant repA gene, a kanamycin resistant gene, and a levansucrase gene SacB derived from *Bacillus subtilis.* The levansucrase gene lethally acts on a host microorganism in the presence of sucrose. The PCR fragment was amplified using PrimeSTAR Max DNA Polymerase (trade name, manufactured by Takara Bio Inc.), and the plasmid was prepared using an *Escherichia coli* HST08 strain. Using the genomic DNA of the *Escherichia coli* BL21 (DE3) strain as a template, a PCR product containing an upstream region, a coding region, and a downstream region of a disruption target gene was obtained. The combinations of the target gene and the primer sequence are shown in Table 5.

**[Table 5]**

| Target gene | Forward primer | | Reverse primer | |
|---|---|---|---|---|
| | Base sequence | SEQ ID NO: | Base sequence | SEQ ID NO: |
| yqhD | TTGTTGAATAAATCGTCTG TTTGCCGAGAATACGCG | 77 | TATCGCATGCAGATCTGCGCT AATGGGCTCCAGGA | 78 |
| fucO | TTGTTGAATAAATCGCGCG CCAGCGCTGGCTGTTT | 79 | TATCGCATGCAGATCCCGCCA ATGCCGGAAGAGTT | 80 |
| adhP | TTGTTGAATAAATCGCGAT CGTGATGCCGCTGTCT | 81 | TATCGCATGCAGATCGACAAC GTAGGCTTTGTTCA | 32 |
| eutG | TTGTTGAATAAATCGCGCC ATCTCGACACTCTTGA | 83 | TATCGCATGCAGATCGCGAAC ATCGATGGGTTAGC | 84 |
| ybbO | TTGTTGAATAAATCGTGGC ATTTGCCCTTCCTGTT | 85 | TATCGCATGCAGATCGTCCTG ATCCTGCAACGGAA | 86 |
| ahr | TTGTTGAATAAATCGCTCA TAACGGTACTGCAAAC | 87 | TATCGCATGCAGATCTCGCAG CAGGTAAGATGATT | 88 |
| yahK | TTGTTGAATAAATCGATAT TCGTCCTAACGAACAG | 89 | TATCGCATGCAGATCTTTTTG ATTTTCAAGTATGT | 90 |

Next, the PCR product was inserted into the pHAK1 plasmid fragment amplified using primers 7 and 8 with InFusion (registered trademark) HD cloning kit (trade name, manufactured by Clontech Laboratories, Inc.) and the PCR product was circularized. The sequences of the primers used are shown in Table 6.

**[Table 6]**

| Primer No. | Base sequence | SEQ ID NO: |
|---|---|---|
| 7 | GATCTGCATGCGATATCTCTAGAACGCGTAAGCTT | 91 |
| 8 | TCTCGAGCCGATTTATTCAACAAAGCCGC | 92 |

PCR was performed using the pHAK1 plasmid into which the DNA fragment containing the upstream region, the coding region, and the downstream region of the resulting disruption target gene as a template and using primers shown in Table 7, thereby obtaining a plasmid fragment in which the coding region of the disruption target gene was partially or entirely removed.

**[Table 7]**

| Target gene | Forward primer | | Reverse primer | |
|---|---|---|---|---|
| | Base sequence | SEQ ID NO: | Base sequence | SEQ ID NO: |
| yqhD | ACTTTCGTTTTCGGGCATT TCGTCC | 93 | CCAATATGAGGGCAGAGAAC GATC | 94 |
| fucO | ATGCGCTGATGTGATAATG C | 95 | CCTTCTCCTTGTTGCTTTA | 96 |
| adhP | GAGGCCTTTGCTGCGACT GC | 97 | AGTTCCTCCTTTTCGGATGA T | 98 |
| eutG | ATGCCGGATGCGACGCTT | 99 | TCATTTTGCATATAGCCCCT | 100 |
| ybbO | TGACCTGGGCAGTAATGG TG | 101 | CAGGATCTCCGTTGCTTTAT GAGTC | 102 |
| ahr | CGTGGTGTTGAAAGCCGA TTATTG | 103 | CATAAACTTCCAGTTCTCCG CCC | 104 |
| yahK | TCGCACACTAACAGACTGA A | 105 | TGTGTTTACTCCTGATTAGC | 106 |

The resulting plasmid fragment was circularized by terminal phosphorylation and self-ligation to obtain a plasmid for disrupting a gene.

### (Construction of ADH Gene-Disrupted Escherichia coli Strain)

An *Escherichia coli* BL21 (DE3) strain was transformed with a plasmid for destruction of a desired gene by a calcium chloride method and then applied to an LB agar medium containing 100 mg/L of kanamycin sulfate, and culture was performed at 30°C overnight, thereby obtaining a transformant. The present transformant was inoculated, with a platinum loop, into 1 mL of an LB medium containing 100 mg/L of kanamycin sulfate, and shaking culture was performed at 30°C. The resulting culture medium was applied to an LB agar medium containing 100 mg/L of kanamycin sulfate, and culture was performed at 42°C overnight. In the resulting colony, a plasmid was inserted into the genome by single crossover. The colony was inoculated into 1 mL of an LB liquid medium with a platinum loop, and shaking culture was performed at 30°C. The resulting culture medium was applied to an LB agar medium containing 10% sucrose, and culture was performed overnight. Disruption of a desired gene in the resulting colony was observed by colony direct PCR using the primer set shown in Table 8. ADH-deficient *Escherichia coli* in which the ADH7 gene was disrupted was constructed from the above operation.

**[Table 8]**

| Target gene | Forward primer | | Reverse primer | |
|---|---|---|---|---|
| | Base sequence | SEQ ID NO: | Base sequence | SEQ ID NO: |
| yqhD | TATTCTCAATCCGTT TCAGCACGCG | 107 | TTCGGGATCACCAC CAGGCCG | 108 |
| fucO | CGGAAATGGACGAAC AGTGG | 109 | CGTCATCAGCGTTT ACCAGATT | 110 |
| adhP | GCATAAACACTGTCC GCGTC | 111 | GAAATCGAGAAGGC AGAAGCGAAA | 112 |
| eutG | GGTGCGGTCACCATT[?] GTTCG | 113 | CATATCGCACGCCA GCAGTG | 114 |
| ybbO | GGTGAGGATGGAGA GTTCATG | 115 | CAGTTCGATTTGCG CCACCAG | 116 |
| ahr | TCCGCTAGTGTGATT TCAGG | 117 | GAAATTATTATGCC GCCAGGCGT | 118 |
| yahK | TTATGGTCTGGGCGA CATGC | 119 | GCATCATCCTGGTC ATATACCC | 120 |

### (Enzyme Reaction)

Using succinyl-CoA as a substrate, studies were conducted on (1) converting 4-oxobutanoic acid produced by the enzyme reaction according to the present invention into 4-hydroxybutanoic acid by an alcohol dehydrogenase or (2) converting 4-oxobutanoic acid into 4-aminobutanoic acid by an aminotransferase.

The ADH-deficient *Escherichia coli* strain was transformed using any one of a wild-type enzyme expression plasmid, a modified enzyme expression plasmid, an alcohol dehydrogenase expression plasmid, and an empty plasmid containing no enzyme gene.

The colonies were inoculated into an LB medium (15 m tube, liquid volume 2 mL), and culture was performed at 30°C by shaking overnight. Each 30 uL of the culture solution was diluted 10-fold and OD600 values were measured and recorded with Tecan Infinite 200 microplate reader. The remaining culture solution was transferred to a 2 mL tube, and centrifugation was performed at 8,000 rpm for 3 minutes with a Centrifuge 5424R tabletop micro centrifuge manufactured by Eppendorf SE. The supernatant was discarded, the pellets were resuspended in a 100 mM Tris-HCl buffer (pH = 8.0) to show OD600 = 30, and then bacterial cells were disrupted with an ultrasonic crusher. The disrupted solution was centrifuged at 13,200 rpm for 15 minutes, and the supernatant was used as an enzyme solution. The enzyme reaction was performed in a 1.5 mL tube. The composition of the reaction solution is as follows.

Reaction solution 1: modified aldehyde dehydrogenase 5 µL, aminotransferase 20 µL, total amount 200 µL, final concentration 2 mM succinyl-CoA, 4 mM sodium glutamate, 8 mM NADPH, 0.01 mM pyridoxal phosphate, 100 mM Tris-HCl buffer pH = 8.0.

Reaction solution 2: modified aldehyde dehydrogenase 5 µL, alcohol dehydrogenase 20 µL, total amount 200 µL, final concentration 2 mM succinyl-CoA, 8 mM NADPH, 100 mM Tris-HCl buffer pH = 8.0.

The prepared tube was shaken at 30°C and 1,000 rpm for 4 hours with a tabletop shaker (M·BR-022UP) manufactured by TAITEC Corporation. The sample after the reaction was diluted 4-fold with 10 mM MSA and analyzed by the analysis method shown in Table 4.

The analysis results are shown in Tables 9-1 and 9-2. The values are mean values of two independent assays. It was found that the amounts of 4-hydroxybutanoic acid and 4-aminobutanoic acid produced were reduced in the sample to which the mutant enzyme in which the NADPH consumption rate was reduced when succinyl-CoA was used as a substrate was added in <2> above, as compared with the sample to which the wild-type enzyme was added. In particular, when I250F mutant was used, the succinyl-CoA reaction was suppressed to such an extent that it was comparable to a sample to which no acyl-CoA reductase was added (empty vector).

**[Table 9-1]**

| | | 4-hydroxybutyrate (mM) |
|---|---|---|
| Comparative Example | Wild-type | 1.17 |
| Example | I250F | 0.19 |
| Example | I250A | 0.52 |
| Comparative Example | Empty vector | 0.16 |

**[Table 9-2]**

| | | 4-aminobutyrate (µM) |
|---|---|---|
| Comparative Example | Wild-type | 14.27 |
| Example | I250F | 0.87 |
| Example | I250A | 5.44 |
| Comparative Example | Empty vector | 0.63 |

### <5> Evaluation of Activity of Double-Mutant Enzyme

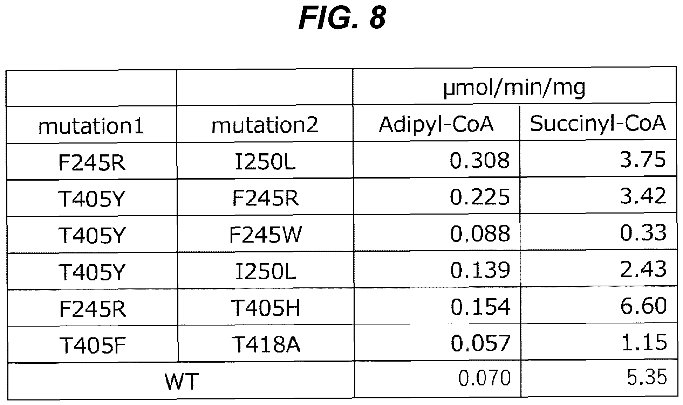

A substrate and a double-mutant enzyme were prepared in the same manner as the procedure shown in "<3> Evaluation of Activity of Point Mutant Enzymes for Various Substrates" above, and the enzyme activity was evaluated. The activity of the mutant enzyme for each substrate is illustrated in Fig. 8. By introducing a plurality of mutations, it was possible to reduce the succinyl-CoA activity and to obtain a mutant enzyme in which the adipyl-CoA activity was improved.

### Industrial Applicability

The present invention can be used for efficient fermentation production including an acyl-CoA reduction reaction, and is expected to be applied to produce a target compound on an industrial scale.

## Claims

1. A recombinant polypeptide comprising:
(a) an amino acid sequence A having a sequence identity of 60% or higher, 65% or higher, 70% or higher, 75% or higher, 80% or higher, 85% or higher, 88% or higher, 90% or higher, 93% or higher, 95% or higher, 97% or higher, 98% or higher, or 99% or higher with an amino acid sequence set forth in SEQ ID NO: 1;
(b) a substitution of at least one amino acid at a position corresponding to a substrate-binding site of a polypeptide having an amino acid sequence set forth in SEQ ID NO: 1 in the amino acid sequence A; and
(c) a reducing activity R of converting CoA thioester of an acyl-CoA compound into an aldehyde group,
wherein the reducing activity R includes:
(c-1) a reducing activity R1 of converting adipyl-CoA into 5-formylpentanoic acid in one step; and
(c-2) a reducing activity R2 of converting succinyl-CoA into succinic semialdehyde.

2. The recombinant polypeptide according to claim 1, wherein the reducing activity R2 of (c-2) is lower than a reducing activity R2' of the polypeptide having the amino acid sequence set forth in SEQ ID NO: 1 that converts succinyl-CoA into succinic semialdehyde.

3. The recombinant polypeptide according to claim 1 or 2, wherein the recombinant polypeptide is selected from the group of succinic semialdehyde dehydrogenase (EC 1.2.1.76).

4. The recombinant polypeptide according to any one of claims 1 to 3, wherein the recombinant polypeptide is derived from a microorganism of the genus *Clostridium.*

5. The recombinant polypeptide according to any one of claims 1 to 4, wherein the position corresponding to the substrate-binding site of the polypeptide having the amino acid sequence set forth in SEQ ID NO: 1 in (b) is a position corresponding to positions 75, 78, 79, 245, 250, 252, 405, 406, 410, 418, 419, 420, and 926 based on the amino acid sequence set forth in SEQ ID NO: 1.

6. The recombinant polypeptide according to any one of claims 1 to 5, wherein the reducing activity R1 of (c-1) is improved compared to a reducing activity R1' of the polypeptide having the amino acid sequence set forth in SEQ ID NO: 1 that converts adipyl-CoA into 5-formylpentanoic acid in one step.

7. The recombinant polypeptide according to any one of claims 1 to 6, wherein at least two amino acids at the positions corresponding to the substrate-binding site of the polypeptide having the amino acid sequence set forth in SEQ ID NO: 1 in (b) are substituted in the amino acid sequence A.

8. The recombinant polypeptide according to any one of claims 1 to 7, wherein in the amino acid sequence A, at least one of the following (i) to (xii) is satisfied based on the amino acid sequence set forth in SEQ ID NO: 1:
(i) an amino acid residue at a position corresponding to position 75 is substituted with any of alanine and phenylalanine;
(ii) an amino acid residue at a position corresponding to position 78 is substituted with any of alanine and phenylalanine;
(iii) an amino acid residue at a position corresponding to position 79 is substituted with any of alanine and phenylalanine;
(iv) an amino acid residue at a position corresponding to position 245 is substituted with any of alanine, phenylalanine, arginine, glutamic acid, serine, asparagine, glutamine, glycine, leucine, and tryptophan;
(v) an amino acid residue at a position corresponding to position 250 is substituted with any of alanine, phenylalanine, lysine, arginine, histidine, threonine, asparagine, glutamine, glycine, leucine, valine, proline, and tryptophan;
(vi) an amino acid residue at a position corresponding to position 252 is substituted with any of alanine and phenylalanine;
(vii) an amino acid residue at a position corresponding to position 405 is substituted with any of alanine, phenylalanine, lysine, histidine, glutamic acid, serine, asparagine, glutamine, leucine, valine, tyrosine, proline, and tryptophan;
(viii) an amino acid residue at a position corresponding to position 406 is substituted with any of alanine and phenylalanine;
(ix) an amino acid residue at a position corresponding to position 410 is substituted with phenylalanine;
(x) an amino acid residue at a position corresponding to position 418 is substituted with any of alanine and phenylalanine;
(xi) an amino acid residue at a position corresponding to position 419 is substituted with any of alanine and phenylalanine; and
(xii) an amino acid residue at a position corresponding to position 420 is substituted with any of alanine and phenylalanine.

9. The recombinant polypeptide according to any one of claims 1 to 8, wherein in the amino acid sequence A, at least one of the following (i) to (xii) is satisfied based on the amino acid sequence set forth in SEQ ID NO: 1:
(i) an amino acid residue at a position corresponding to position 75 is substituted with any of alanine and phenylalanine;
(ii) an amino acid residue at a position corresponding to position 78 is substituted with any of alanine and phenylalanine;
(iii) an amino acid residue at a position corresponding to position 79 is substituted with alanine;
(iv) an amino acid residue at a position corresponding to position 245 is substituted with any of alanine, phenylalanine, arginine, glutamic acid, serine, asparagine, glutamine, glycine, leucine, and tryptophan;
(v) an amino acid residue at a position corresponding to position 250 is substituted with any of alanine, phenylalanine, lysine, arginine, histidine, threonine, asparagine, glutamine, glycine, leucine, valine, proline, and tryptophan;
(vi) an amino acid residue at a position corresponding to position 252 is substituted with any of alanine and phenylalanine;
(vii) an amino acid residue at a position corresponding to position 405 is substituted with any of alanine, phenylalanine, lysine, histidine, glutamic acid, serine, asparagine, glutamine, leucine, valine, tyrosine, proline, and tryptophan;
(viii) an amino acid residue at a position corresponding to position 406 is substituted with any of alanine and phenylalanine;
(ix) an amino acid residue at a position corresponding to position 410 is substituted with phenylalanine;
(x) an amino acid residue at a position corresponding to position 418 is substituted with alanine;
(xi) an amino acid residue at a position corresponding to position 419 is substituted with alanine; and
(xii) an amino acid residue at a position corresponding to position 420 is substituted with alanine.

10. The recombinant polypeptide according to any one of claims 1 to 9, wherein in the amino acid sequence A, at least one of the following (i) to (xii) is satisfied based on the amino acid sequence set forth in SEQ ID NO: 1:
(i) an amino acid residue at a position corresponding to position 75 is substituted with any of alanine and phenylalanine;
(ii) an amino acid residue at a position corresponding to position 78 is substituted with phenylalanine;
(iii) an amino acid residue at a position corresponding to position 79 is substituted with any of alanine and phenylalanine;
(iv) an amino acid residue at a position corresponding to position 245 is substituted with any of arginine, glutamine, glycine, serine, and tryptophan;
(v) an amino acid residue at a position corresponding to position 250 is substituted with any of alanine, phenylalanine, leucine, and proline;
(vi) an amino acid residue at a position corresponding to position 252 is substituted with any of alanine and phenylalanine;
(vii) an amino acid residue at a position corresponding to position 405 is substituted with any of alanine, phenylalanine, lysine, histidine, glutamic acid, serine, glutamine, tyrosine, and tryptophan;
(viii) an amino acid residue at a position corresponding to position 406 is substituted with any of alanine and phenylalanine;
(ix) an amino acid residue at a position corresponding to position 410 is substituted with phenylalanine;
(x) an amino acid residue at a position corresponding to position 418 is substituted with any of alanine and phenylalanine;
(xi) an amino acid residue at a position corresponding to position 419 is substituted with any of alanine and phenylalanine; and
(xii) an amino acid residue at a position corresponding to position 420 is substituted with any of alanine and phenylalanine.

11. The recombinant polypeptide according to any one of claims 1 to 10, wherein in the amino acid sequence A, at least one of the following is satisfied based on the amino acid sequence set forth in SEQ ID NO: 1:
(iv) an amino acid residue at a position corresponding to position 245 is substituted with any of arginine, serine, and tryptophan;
(v) an amino acid residue at a position corresponding to position 250 is substituted with leucine; and
(vii) an amino acid residue at a position corresponding to position 405 is substituted with any of lysine, histidine, serine, glutamine, tyrosine, and tryptophan.

12. The recombinant polypeptide according to any one of claims 1 to 11, wherein in the amino acid sequence A, any one of the following is satisfied based on the amino acid sequence set forth in SEQ ID NO: 1,
the substitution is made at any one of:
· a position corresponding to position 245 and a position corresponding to position 250;
· a position corresponding to position 245 and a position corresponding to position 405;
· a position corresponding to position 250 and a position corresponding to position 405; and
· a position corresponding to position 405 and a position corresponding to position 418.

13. The recombinant polypeptide according to any one of claims 1 to 12, wherein in the amino acid sequence A, any one of the following is satisfied based on the amino acid sequence set forth in SEQ ID NO: 1:
· an amino acid residue at a position corresponding to 245 position is arginine and an amino acid at a position corresponding to 250 position is leucine;
· an amino acid residue at a position corresponding to 245 position is arginine and an amino acid at a position corresponding to 405 position is tyrosine;
· an amino acid residue at a position corresponding to 245 position is tryptophan and an amino acid at a position corresponding to 405 position is tyrosine;
· an amino acid residue at a position corresponding to 245 position is arginine and an amino acid at a position corresponding to 405 position is histidine;
· an amino acid residue at a position corresponding to 250 position is leucine and an amino acid at a position corresponding to 405 position is tyrosine; and
· an amino acid residue at a position corresponding to 418 position is alanine and an amino acid at a position corresponding to 405 position is phenylalanine.

14. A DNA encoding the recombinant polypeptide according to any one of claims 1 to 13.

15. A recombinant plasmid comprising the DNA according to claim 14.

16. A recombinant microorganism into which the DNA according to claim 14 is introduced.

17. The recombinant microorganism according to claim 16, wherein the recombinant microorganism has an adipyl-CoA production ability.

18. A production method of a target compound, the production method comprising converting adipyl-CoA into 5-formylpentanoic acid in the presence of the recombinant polypeptide according to any one of claims 1 to 14, or a culture of the recombinant microorganism according to claim 16 or 17 and/or an extract of the culture,
wherein the target compound is selected from the group consisting of 5-formylpentanoic acid, 6-aminocaproic acid, 1,6-diaminohexane, 6-hydroxyhexanoic acid, hexanedial, 6-aminohexanal, 6-hydroxyhexanal, and 1,6-hexanediol.

19. The production method according to claim 18, further comprising converting the 5-formylpentanoic acid into 6-aminocaproic acid,
wherein the target compound is 6-aminocaproic acid.

20. The production method according to claim 18, further comprising converting the 5-formylpentanoic acid into 6-hydroxyhexanoic acid,
wherein the target compound is 6-hydroxyhexanoic acid.

21. The production method according to claim 18, further comprising:
i) converting the 5-formylpentanoic acid into 6-hydroxyhexanoic acid,
converting the 6-hydroxyhexanoic acid into 6-hydroxyhexanal, and
converting the 6-hydroxyhexanal into 1,6-hexanediol; or
ii) converting the 5-formylpentanoic acid into hexanedial,
converting the hexanedial into 6-hydroxyhexanal, and
converting the 6-hydroxyhexanal into 1,6-hexanediol,
wherein the target compound is 1,6-hexanediol.

22. The production method according to claim 18, further comprising:
i) converting the 5-formylpentanoic acid into 6-aminocaproic acid,
converting the 6-aminocaproic acid into 6-aminohexanal, and
converting the 6-aminohexanal into 1,6-diaminohexane; or
ii) converting the 5-formylpentanoic acid into hexanedial,
converting the hexanedial into 6-aminohexanal, and
converting the 6-aminohexanal into 1,6-diaminohexane,
in which the target compound is 1,6-diaminohexane.

23. The production method according to claim 19 or 22, wherein the converting of the 5-formylpentanoic acid into 6-aminocaproic acid, the converting of the hexanedial into 6-aminohexanal, and the converting of the 6-aminohexanal into 1,6-diaminohexane are catalyzed by an enzyme selected from the group consisting of:
· 4-aminobutanoate-2-oxoglutarate transaminase (EC 2.6.1.19);
· putrescine-2-oxoglutarate transaminase (EC 2.6.1.82);
· 4-aminobutanoate-pyruvate transaminase (EC 2.6.1.96); and
· putrescine-pyruvate transaminase (EC 2.6.1.113).

24. The production method according to claim 23, wherein the enzyme is derived from a microorganism selected from the group consisting of the genus *Vibrio* and the genus *Escherichia.*

25. The production method according to claim 21 or 22, wherein the converting of the 6-hydroxyhexanoic acid into 6-hydroxyhexanal, the converting of the 5-formylpentanoic acid into hexanedial, and the converting of the 6-aminocaproic acid into 6-aminohexanal are catalyzed by an enzyme belonging to a carboxylate reductase (EC 1.2.1.30).

26. The production method according to claim 25, wherein the carboxylate reductase is derived from a microorganism selected from the group consisting of the genus *Mycobacterium* and the genus *Nocardia.*

27. The production method according to claim 20 or 21, wherein the converting of the 5-formylpentanoic acid into 6-hydroxyhexanoic acid, the converting of the hexanedial into 6-hydroxyhexanal, and the converting of the 6-hydroxyhexanal into 1,6-hexanediol are catalyzed by an enzyme selected from the group consisting of:
· alcohol dehydrogenase (EC 1.1.1.1); and
· alcohol dehydrogenase (EC 1.1.1.2).

28. The production method according to claim 27, wherein the alcohol dehydrogenase is derived from a microorganism selected from the group consisting of the genus *Escherichia* and the genus *Bacillus.*

29. A recombinant microorganism comprising:
a pathway for producing at least one selected from the group consisting of 5-formylpentanoic acid, 6-aminocaproic acid, 1,6-diaminohexane, 6-hydroxyhexanoic acid, hexanedial, 6-aminohexanal, 6-hydroxyhexanal, and 1,6-hexanediol; and
an exogenous nucleic acid sequence encoding an enzyme having an amino acid sequence set forth in SEQ ID NO: 1 or an enzyme having an amino acid sequence having a sequence identity of 60% or higher, 65% or higher, 70% or higher, 75% or higher, 80% or higher, 85% or higher, 88% or higher, 90% or higher, 93% or higher, 95% or higher, 97% or higher, 98% or higher, or 99% or higher with the amino acid sequence set forth in SEQ ID NO: 1 and having a reducing activity R of converting CoA thioester of an acyl-CoA compound into an aldehyde group.

30. A recombinant polypeptide comprising:
(a) an amino acid sequence A having a sequence identity of 85% or higher, 88% or higher, 90% or higher, 93% or higher, 95% or higher, 97% or higher, 98% or higher, or 99% or higher with an amino acid sequence set forth in SEQ ID NO: 1;
(b) a substitution of at least one amino acid at a position corresponding to a substrate-binding site of a polypeptide having an amino acid sequence set forth in SEQ ID NO: 1 in the amino acid sequence A; and
(c) a reducing activity R of converting CoA thioester of an acyl-CoA compound into an aldehyde group.
